# EUROPEAN PATENT SPECIFICATION

(11) **EP 0 652 953 B1**
(45) Date of publication and mention of the grant of the patent: **26.01.2005**
(21) Application number: 93918529.4
(22) Date of filing: 29.07.1993
(51) Int. Cl.: C12N 15/12, A61K 38/00, G01N 33/50, G01N 33/53

(54) **MORPHOGENIC PROTEIN SOLUBLE COMPLEX AND COMPOSITION THEREOF**
LÖSLICHER KOMPLEX MORPHOGENER PROTEINE UND ZUSAMMENSETZUNGEN DAVON
COMPLEXE SOLUBLE DE PROTEINES MORPHOGENIQUES ET SA COMPOSITION

(30) Priority: 31.07.1992 US 923780; 04.03.1993 US 29335; 31.03.1993 US 40510
(43) Date of publication of application: 17.05.1995
(73) Proprietor: STRYKER CORPORATION, Kalamazoo, MI 49001 (US)
(72) Inventor: JONES, William, K., Brookline, MA 02116 (US); TUCKER, Ronald, F., Holliston, MA 01746 (US); RUEGER, David, C., Hopkinton, MA 01748 (US); OPPERMANN, Hermann, Medway, MA 02053 (US); OZKAYNAK, Engin, Milford, MA 01757 (US); KUBERASAMPATH, Thangavel, Medway, MA 02053 (US)
(74) Representative: Price, Vincent Andrew
(86) International application number: PCT/US1993/007189
(87) International publication number: WO 1994/003600

(56) References cited:
- WO-A-91/18047
- WO-A-92/07073
- WO-A-93/05751
- WO-A-94/20539
- US-A- 4 857 456
- MOLECULAR ENDOCRINOLOGY vol. 5, no. 1 , January 1991 pages 149 - 155 R. GLENN HAMMONDS, JR. ET AL. 'Bone-inducing activity of mature BMP-2b produced from a hybrid BMP-2a/2b precursor'

## Description

### Field of the Invention

The present invention relates generally to morphogenic proteins and, more particularly, to compositions having improved solubility in aqueous solvents.

### Background of the Invention

Morphogenic proteins ("morphogens") are well known and described in the art. See, for example, U.S. Pat. Nos. 4, 968,590; 5,011,691; 5,018,753; PCT US92/01968 and PCT US92/07432; as well as various articles published in the scientific literature, including Ozkaynak et al. (1992) J.Biol. Chem. 267:25220-25227 and Ozkaynak et al. (1991) Biochem. Biophys. Res. Comm. 179:116-123. The art has described how to isolate morphogenic proteins from bone, how to identify genes encoding these proteins and how to express them using recombinant DNA technology. The morphogenic proteins are capable of inducing endochondral bone formation and other tissue formation in a mammal when they are properly folded, dimerized and disulfide bonded to produce a dimeric species having the appropriate three dimensional conformation. The proteins have utility in therapeutic applications, either by direct or systemic administration. Where bone induction is desired, for example, the morphogen typically is provided to the desired site for bone formation in a mammal in association with a suitable matrix having the appropriate conformation to allow the infiltration, proliferation and differentiation of migrating progenitor cells. The morphogenic protein adsorbed to the surfaces of a suitable matrix is generally referred to in the art as an osteogenic device. The proteins can be isolated from bone or, preferably, the gene encoding the protein is produced recombinantly in a suitable host cell.

The morphogen precursor polypeptide chains share a common structural motif, including a N-terminal signal sequence and pro region, both of which are cleaved to produce a mature sequence, capable of disulfide bonding and comprising an N-terminal extension and a C-terminal domain whose amino acid sequence is highly conserved among members of the family. In their mature dimeric forms, the morphogens typically are fairly insoluble under physiological conditions. Increasing the solubility of these proteins has significant medical utility as it would enhance systemic administration of morphogens as therapeutics. Various carrier proteins, including serum albumin and casein are known to increase the solubility of morphogens (see, for example, PCT US92/07432). PCT US92/05309 (WO 93/00050) discusses the use of various solubilizing agents, including various amino acids and methyl esters thereof, as well as guanidine, sodium chloride and heparin, to increase the solubility of mature dimeric BMP2.

Improved methods for the recombinant expression of morphogenic proteins is an ongoing effort in the art. For example, Hammonds et al. (1991) Mol. Endocrin. 4:149-155 and WO 91/18047 disclose the construction of recombinant BMP 2a/BMP-2b hybrid genes and their expression in mammalian cell culture. It was found that the expression of BMP-2b mature protein may be enhanced by ligating a DNA sequence encoding the BMP-2a pro region upstream of the mature BMP-2b DNA sequence.

It is object of this invention to provide an improvement in the methods for producing and purifying morphogenic proteins having high specific activity, and for formulating compositions and osteogenic devices comprising these proteins. Another object is to provide soluble forms of morphogenic proteins consisting essentially of amino acid sequences derived from morphogenic proteins. Another object is to provide formulations which stabilize the soluble complex of morphogenic proteins. Still another object is to provide means for distinguishing between soluble forms of the protein and the mature morphogenic species, to provide means for quantitating the amounts of these proteins in a fluid, including a body fluid, such as serum, cerebro-sprinal fluid or peritoneal fluid, and to provide polyclonal and monoclonal antibodies capable of distinguishing between these various species.

Another object is to provide antibodies and biological diagnostic assays for monitoring the concentration of morphogens and endogenous anti-morphogen antibodies present in a body fluid and to provide kits and assays for detecting fluctuations in the concentrations of these proteins in a body fluid. U.S. Patent No. 4,857,456 and Urist et al. (1984) Proc. Soc. Exp. Biol. Med. 176:472-475 describe a serum assay for detecting a protein purported to be a bone morphogenetic protein. The protein is not a member of the morphogen family of proteins described herein, differing in molecular weight, structural characteristics and solubility from these proteins.

### Summary of the Invention

It now has been discovered that morphogenic protein secreted into cultured medium from mammalian cells contains as a significant fraction of the secreted protein a soluble form of the protein, and that this soluble form comprises the mature dimeric species, including truncated forms thereof, noncovalently associated with at least one, and preferably two pro domains. It further has been discovered that antibodies can be used to discriminate between these two forms of the protein. These antibodies may be used as part of a purification scheme to selectively isolate the mature or the soluble form of morphogenic protein, as well as to quantitate the amount of mature and soluble forms produced. These antibodies also may be used as part of diagnostic treatments to monitor the concentration of morphogenic proteins in solution in a body and to detect fluctuations in the concentration of the proteins in their various forms. The antibodies and proteins also may be used in diagnostic assays to detect and monitor concentrations of endogenous anti-morphogen antibodies to the various forms of these proteins in the body.

An important embodiment of the invention is a dimeric protein comprising a pair of polypeptide subunits associated to define a dimeric structure having morphogenic activity. As defined herein and in parent, related applications, morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells.

Each of the subunits of the dimeric morphogenic protein comprises at least the 100 amino acid peptide sequence having at least 70% amino acid sequence homology with the C-terminal seven cysteine domain of human OP-1, residues 330-431 of Seq. .ID No. 1. At least one of the subunits comprises the mature form of a subunit of a member of the morphogen family, or an amino acid sequence variant thereof, noncovalently complexed with a peptide consisting of a pro-region selected from sequences defined by residues 30-48, 30-292 and 40-292 of Seq. ID No. 1 a member of the morphogen family, or an allelic, species, chimeric or other sequence variant thereof. The pair of subunits and one or, preferably, two pro region peptides, together form a complex which is more soluble in aqueous solvents than the uncomplexed pair of subunits, said complex eliciting morphogenic activity in vivo..

Preferably, both subunits comprise a mature form of a subunit of a member of the morphogen family or an allelic, species, chimeric or other sequence variant thereof, and both subunits are noncovalently complexed with a peptide comprising a pro region, or a fragment thereof. Most preferably, each subunit is the mature form of human OP-1, or a species, allelic or other sequence variant thereof, and the pro region peptide is the entire or partial sequence of the pro region of human OP-1 as defined above. Currently, the preferred pro region is the full length form of the pro region.

As will be appreciated by those having ordinary skill in the art, useful sequences encoding said pro region may be obtained from genetic sequences encoding known morphogens.

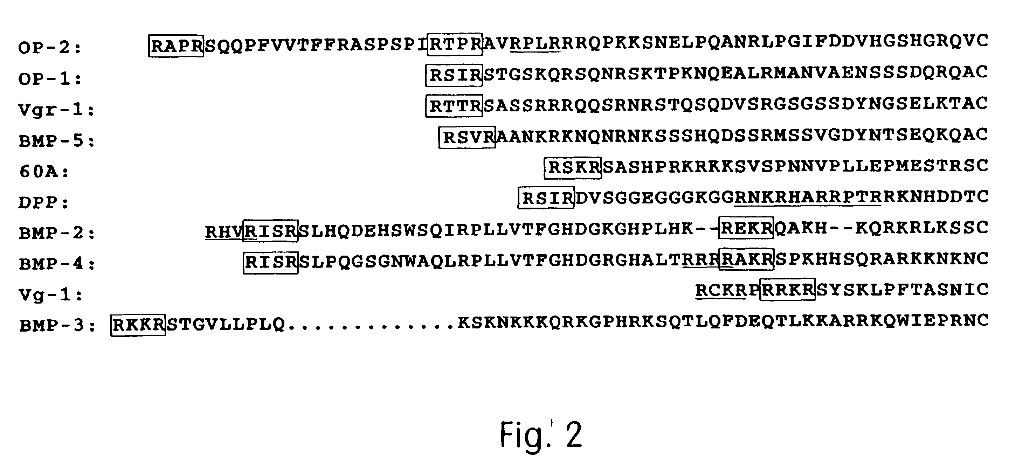

As used herein, the mature form of a morphogen protein subunit includes the intact C-terminal domain and intact or truncated forms of the N-terminal extensions. For example, useful mature forms of OP-1 include dimeric species defined by residues 293-431 of Seq ID No. 1, as well as truncated sequences thereof, including sequences defined by residues 300-431, 313-431, 315-431, 316-431 and 318-431. Note that this last sequence retains only about the last 10 residues of the N-terminal extension sequence. Fig. 2 presents the N-terminal extensions for a number of preferred morphogen sequences. Canonical Arg-Xaa-Xaa-Arg cleavage sites where truncation may occur are boxed or underlined in the figure. As will be appreciated by those having ordinary skill in the art, mature dimeric species may include subunit combinations having different N-terminal truncations.

Other soluble forms of morphogens include dimers of the uncleaved pro forms of these proteins (see below), as well as "hemi-dimers" wherein one subunit of the dimer is an uncleaved pro form of the protein, and the other subunit comprises the mature form of the protein, including truncated forms thereof, noncovalently complexed with a cleaved pro domain.

The soluble proteins of this invention also are useful in the formation of therapeutic compositions for administration to a mammal, particularly a human, and for the development of biological assays for monitoring the concentration of these proteins and endogenous antibodies to these proteins in cell samples and body fluids, including, but not limited to, serum, cerebrospinal fluid and peritoneal fluid.

The foregoing and other objects, features and advantages of the present invention will be made more apparent from the following detailed description of the invention.

### Brief Description of the Drawings

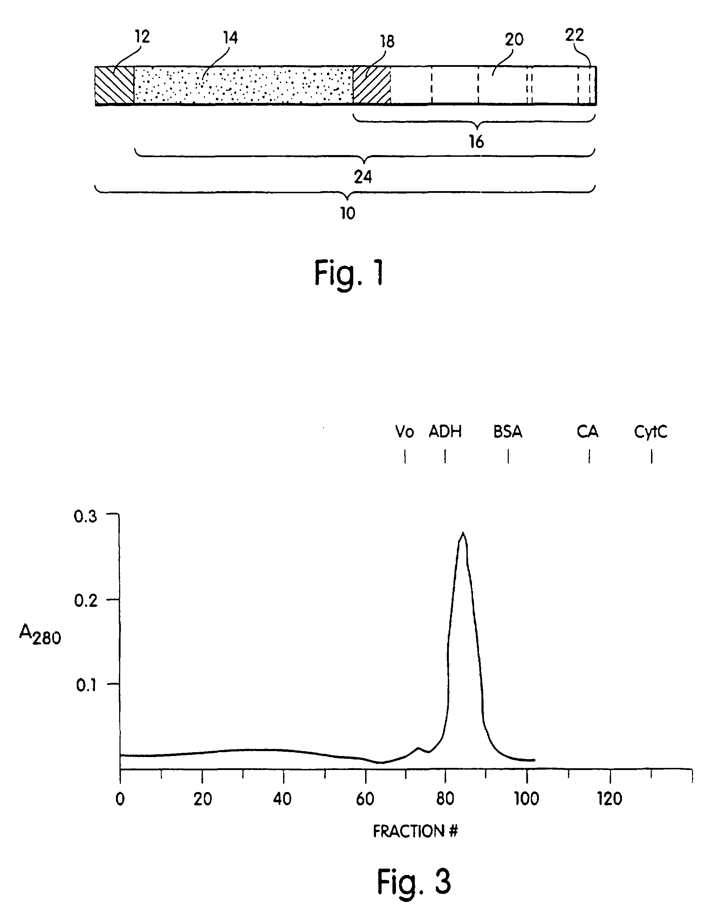

Fig. 1 is a schematic representation of a morphogen polypeptide chain as expressed from a nucleic acid encoding the sequence, wherein the cross-hatched region represents the signal sequence; the stippled region represents the pro domain; the hatched region represents the N-terminus ("N-terminal extension") of the mature protein sequence; and the open region represents the C-terminal region of the mature protein sequence defining the conserved seven cysteine domain, the conserved cysteines being indicated by vertical hatched lines;
Fig.2 lists the sequences of the N-terminal extensions of the mature forms of various morphogens; and
Fig. 3 is a gel filtration column elution profile of a soluble morphogen (OP-1) produced and purified from a mammalian cell culture by IMAC, S-Sepharose and S-200HR chromatography in TBS (Tris-buffered saline), wherein Vₒ is the void volume, ADH is alcohol dehydrogenase (MW 150 kDa), BSA is bovine serum albumin (MW 67 kDa), CA is carbonic anhydrase (MW 29kDa) and CytC is cytochrome C (MW 12.5 kDa).

### Detailed Description

A soluble form of morphogenic proteins now has been discovered wherein the proteins consist essentially of the amino acid sequence of the protein. The soluble form is a non-covalently associated complex comprising the pro domain or a fragment thereof, noncovalently associated or complexed with a dimeric protein species having morphogenic activity, each polypeptide of the dimer having less than 200 amino acids and comprising at least the C-terminal six, and preferably seven cysteine skeleton defined by residues 335-431 and 330-431, respectively, of Seq. ID No. 1. Preferably, the polypeptide chains of the dimeric species comprise the mature forms of these sequences, or truncated forms thereof. Preferred truncated forms comprise the intact C-terminal domain and at least 10 amino acids of the N-terminal extension sequence. The soluble forms of these morphogenic proteins may be isolated from cultured cell medium, a mammalian body fluid, or may be formulated in vitro.

In vivo, under physiological conditions, the pro domain may serve to enhance the transportability of the proteins, and/or to protect the proteins from proteases and scavenger molecules, including antibodies. The pro domains also may aid in targeting the proteins to a particular tissue and/or to present the morphogen to a morphogen cell surface receptor by interaction with a co-receptor molecule. The isolated proteins may be used in therapeutic formulations, particularly for oral or parenteral administration, and in the development of diagnostic and other tissue evaluating kits and assays to monitor the level of endogenous morphogens and endogenous anti-morphogen antibodies.

Detailed descriptions of the utility of these morphogens in therapies to regenerate lost or damaged tissues and/or to inhibit the tissue destructive effects of tissue disorders or diseases, are provided in international applications US92/01968 (WO92/15323); US92/07358 (WO93/04692) and US92/07432 (WO93/05751). Morphogens, including the soluble morphogen complexes of this invention, are envisioned to have particular utility as part of therapies for regenerating lost or damaged bone, dentin, periodontal, liver, cardiac, lung and nerve tissue, as well as for protecting these tissues from the tissue destructive effects associated with an immunological response. The proteins also are anticipated to provide a tissue protective effect in the treatment of metabolic bone disorders, such as osteoporosis, osteomalacia and osteosarcoma; in the treatment of liver disorders, including cirrhosis, hepatitis, alcohol liver disease and hepatic encephalopathy; and in the treatment or prevention of ischemia reperfusion-associated tissue damage, particularly to nerve or cardiac tissue.

Presented below are detailed descriptions of useful soluble morphogen complexes of this invention, as well as how to make and use them.

### I. Useful Soluble Morphogen Complexes - Protein Considerations

Among the morphogens useful in this invention are proteins originally identified as osteogenic proteins, such as the OP-1, OP-2 and CBMP2 proteins, as well as amino acid sequence-related proteins such as DPP (from Drosophila), Vgl (from Xenopus), Vgr-1 (from mouse, see U.S. 5,011,691 to Oppermann et al.), GDF-1 (from mouse, see Lee (1991) PNAS 88:4250-4254), 60A protein (from Drosophila, Seq. ID No. 24, see Wharton et al. (1991) PNAS 88:9214-9218), and the recently identified OP-3.

The members of this family, which are a subclass of the TGF-β super-family of proteins, share characteristic structural features, represented schematically in Fig. 1, as well as substantial amino acid sequence homology in their C-terminal domains, including a conserved seven cysteine structure. As illustrated in the figure, the proteins are translated as a precursor polypeptide sequence 10, having an N-terminal signal peptide sequence 12, (the "pre pro" region, indicated in the figure by cross-hatching), typically less than about 30 residues, followed by a "pro" region 14, indicated in the figure by stippling, and which is cleaved to yield the mature sequence 16. The mature sequence comprises both the conserved C-terminal seven cysteine domain 20, and an N-terminal sequence 18, referred to herein as an N-terminal extension, and which varies significantly in sequence between the various morphogens. Cysteines are represented in the figure by vertical hatched lines 22. The polypeptide chains dimerize and these dimers typically are stabilized by at least one interchain disulfide bond linking the two polypeptide chain subunits.

The signal peptide is cleaved rapidly upon translation, at a cleavage site that can be predicted in a given sequence using the method of Von Heijne ((1986) Nucleic Acids Research 14:4683-4691.) The "pro" form of the protein subunit, 24, in Fig. 1, includes both the pro domain and the mature domain, peptide bonded together. Typically, this pro form is cleaved while the protein is still within the cell, and the pro domain remains noncovalently associated with the mature form of the subunit to form a soluble species that appears to be the primary form secreted from cultured mammalian cells. Typically, previous purification techniques utilized denaturing conditions that disassociated the complex.

Other soluble forms of morphogens secreted from mammalian cells include dimers of the pro forms of these proteins, wherein the pro region is not cleaved from the mature domain, and "hemi-dimers", wherein one subunit comprises a pro form of the polypeptide chain subunit and the other subunit comprises the cleaved mature form of the polypeptide chain subunit (including truncated forms thereof), preferably noncovalently associated with a cleaved pro domain.

The isolated pro domain typically has a substantial hydrophobic character, as determined both by analysis of the sequence and by characterization of its properties in solution. The isolated pro regions alone typically are not significantly soluble in aqueous solutions, and require the presence of denaturants, e.g., detergents, urea, guanidine HCl, and the like, and/or one or more carrier proteins. Accordingly, without being limited to any given theory, the non-covalent association of the cleaved pro region with the mature morphogen dimeric species likely involves interaction of a hydrophobic portion of the pro region with a corresponding hydrophobic region on the dimeric species, the interaction of which effectively protects or "hides" an otherwise exposed hydrophobic region of the mature dimer from exposure to aqueous environments, enhancing the affinity of the mature dimer species for aqueous solutions.

Morphogens comprise a subfamily of proteins within the TGF-β superfamily of structurally related proteins. Like the morphogens described herein, TGF-β also has a pro region which associates non-covalently with the mature TGF-β protein form. However, unlike the morphogens, the TGF-β pro region contains numerous cysteines and forms disulfide bonds with a specific binding protein. The TGF-β1 pro domain also is phosphorylated at one or more mannose residues, while the morphogen pro regions typically are not.

Pro domains used in the invention are selected from sequences defined by residues 30-48, 30-292 (full length form) and 48-292 . Soluble OP-1 complex stability is enhanced when the pro region comprises the full length form rather than a truncated form, such as the 48-292 truncated form, in that residues 30-47 show sequence homology to the N-terminal portions of other morphogens, and are believed to have particular utility in enhancing complex stability for all morphogens. Accordingly, currently preferred pro sequences are those encoding the full length form of the pro region.

Table I, below, describes the various preferred morphogens identified to date, including their nomenclature as used herein, the sequences defining the various regions of the subunit sequences, their Seq. ID references, and publication sources for their nucleic acid and amino acid sequences. The mature protein sequences defined are the longest anticipated forms of these sequences. As described above, shorter, truncated forms of these sequences also are contemplated. Preferably, truncated mature sequences include at least 10 amino acids of the N-terminal extension. Fig. 2 lists the N-terminal extensions for a number of the preferred morphogen sequences described below. Arg-Xaa-Xaa-Arg cleavage sites that may yield truncated sequences of the mature subunit form are boxed or underlined in the figure.

**TABLE I**

| | |
|---|---|
| "OP-1" | Refers generically to the group of morphogenically active proteins expressed from part or all of a DNA sequence encoding OP-1 protein, including allelic and species variants thereof, e.g., human OP-1 ("hOP-1"), or mouse OP-1 ("mOP-1".) The cDNA sequences and the amino acids encoding the full length proteins are provided in Seq. Id Nos. 1 and 2 (hOP1) and Seq. ID Nos. 3 and 4 (mOP1.) The mature proteins are defined by residues 293-431 (hOP1) and 292-430 (mOP1), wherein the conserved seven cysteine skeleton is defined by residues 330-431 and 329-430, respectively, and the N-terminal extensions are defined by residues 293-329 and 292-329, respectively. The "pro" regions of the proteins, cleaved to yield the mature, morphogenically active proteins, are defined essentially by residues 30-292 (hOP1) and residues 30-291 (mOP1). |
| | |
| "OP-2" | refers generically to the group of active proteins expressed from part or all of a DNA sequence encoding OP-2 protein, including allelic and species variants thereof, e.g., human OP-2 ("hOP-2") or mouse OP-2 ("mOP-2".) The full length proteins are provided in Seq. ID Nos. 5 and 6 (hOP2) and Seq. ID Nos. 7 and 8 (mOP2.) The mature proteins are defined essentially by residues 264-402 (hOP2) and 261-399 (mOP2), wherein the conserved seven cysteine skeleton is defined by residues 301-402 and 298-399, respectively, and the N-terminal extensions are defined by residues 264-300 and 261-297, respectively. The "pro" regions of the proteins, cleaved to yield the mature, morphogenically active proteins likely are defined essentially by residues 18-263 (hOP2) and residues 18-260 (mOP2). (Another cleavage site also occurs 21 residues upstream for both OP-2 proteins.) |
| | |
| "OP-3" | refers generically to the group of active proteins expressed from part or all of a DNA sequence encoding OP-3 protein, including allelic and species variants thereof, e.g., mouse OP-3 ("mOP-3".) The full length protein is provided in Seq. ID No. 9. The mature protein is defined essentially by residues 261-399 or 264-399, wherein the conserved seven cysteine skeleton is defined by residues 298-399 and the N-terminal extension is defined by residues 264-297 or 261-297. The "pro" region of the protein, cleaved to yield the mature, morphogenically active proteins likely is defined essentially by residues 20-262. |
| | |
| "BMP2/BMP4" | refers to protein sequences encoded by the human BMP2 and BMP4 genes. The amino acid sequence for the full length proteins, referred to in the literature as BMP2A and BMP2B, or BMP2 and BMP4, appear in Seq. ID Nos. 10 and 11, respectively, and in Wozney, et al. (1988) Science 242:1528-1534. The pro domain for BMP2 (BMP2A) likely includes residues 25-248 or 25-282; the mature protein, residues 249-396 or 283-396, of which residues 249-296/283-296 define the N-terminal extension and 295-396 define the C-terminal domain. The pro domain for BMP4 (BMP2B) likely includes residues 25-256 or 25-292; the-mature protein, residues 257-408 or 293-408,of which 257-307/293-307 define the N-terminal extension, and 308-408 define the C-terminal domain. |
| | |
| "DPP" | refers to protein sequences encoded by the Drosophila DPP gene. The amino acid sequence for the full length protein, including the mature form and the pro region, appears in Seq.ID No. 12 and in Padgett, et al (1987) Nature 325: 81-84. The pro domain likely extends from the signal peptide cleavage site to residue 456; the mature protein likely is defined by residues 457-588, where residues 457-586 define the N-terminal extension and 487-588 define the C-terminal domain. |
| | |
| "Vgl" | refers to protein sequences encoded by the Xenopus Vgl gene. The amino acid sequence for the full length protein, including the mature form and the pro region, appears in Seq.ID No. 13 and in Weeks (1987) Cell 51: 861-867. The pro domain likely extends from the signal peptide cleavage site to residue 246; the mature protein likely is defined by residues 247-360, where residues 247-258 define the N-terminal extension, and residues 259-360 define the C-terminal domain. |
| | |
| "Vgr-1" | refers to protein sequences encoded by the murine Vgr-1 gene. The amino acid sequence for the full length protein, including the mature form and the pro region, appears in Seq. ID No. 14 and in Lyons, et al, (1989) PNAS 86: 4554-4558. The pro domain likely extends from the signal peptide cleavage site to residue 299; the mature protein likely is defined by residues 300-438, where residues 300-336 define the N-terminal extension and residues 337-438 define the C-terminus. |
| | |
| "GDF-1" | refers to protein sequences encoded by the human GDF-1 gene. The cDNA and encoded amino sequence for the full length protein is provided in Seq. ID. No. 15 and Lee (1991) PNAS 88:4250-4254. The pro domain likely extends from the signal peptide cleavage site to residue 214; the mature protein likely is defined by residues 215-372, where residues 215-256 define the N-terminal extension and residues 257-372 define the C-terminus. |
| | |
| "60A" | refers to protein sequences encoded by the Drosophila 60A gene. The amino acid sequence for the full length protein appears in Seq. ID No. 16 and in Wharton et al. (1991) PNAS 88:9214-9218) The pro domain likely extends from the signal peptide cleavage site to residue 324; the mature protein likely is defined by residues 325-455, wherein residues 325-353 define the N-terminal extension and residues 354-455 define the C-terminus. |
| | |
| "BMP3" | refers to protein sequences encoded by the human BMP3 gene. The amino acid sequence for the full length protein, including the mature form and the pro region, appears in Seq.ID No. 17 and in Wozney et al. (1988) Science 242: 1528-1534. The pro domain likely extends from the signal peptide cleavage site to residue 290; the mature protein likely is defined by residues 291-472, wherein residues 291-370 define the N-terminal extension and residues 371-472 define the C-terminus. |
| | |
| "BMP5" | refers to protein sequences encoded by the human BMP5 gene. The amino acid sequence for the full length protein, including the mature form and the pro region, appears in Seq.ID No. 18 and in Celeste, et al. (1990) PNAS 87: 9843-9847. The pro domain likely extends from the signal peptide cleavage site to residue 316; the mature protein likely is defined by residues 317-454, where residues 317-352 define the N-terminus and residues 352-454 define the C-terminus. |
| | |
| "BMP6" | refers to protein sequences encoded by the human BMP6 gene. The amino acid sequence for the full length protein, including the mature form and the pro region, appears in Seq. ID No. 16 and in Celeste, et al. (1990) PNAS 87: 9843-5847. The pro domain likely includes extends from the signal peptide cleavage site to residue 374; the mature sequence likely includes residues 375-513, where residues 375-411 define the N-terminus and residues 412-513 define the C-terminus. |

Note that the OP-2 and OP-3 proteins have an additional cysteine residue in the C-terminal region (e.g., see residue 338 in these sequences), in addition to the conserved cysteine skeleton in common with the other proteins in this family. The GDF-1 protein has a four amino acid insert within the conserved skeleton ("Gly-Gly-Pro-Pro") but this insert likely does not interfere with the relationship of the cysteines in the folded structure. In addition, the CBMP2 proteins are missing one amino acid residue within the cysteine skeleton.

The dimeric morphogen species are inactive when reduced, but are active as oxidized homodimers and when oxidized in combination with other morphogens of this invention. Thus, as defined herein, a morphogen useful in a soluble morphogen complex is a dimeric protein comprising a pair of polypeptide chains, wherein each polypeptide chain has less than 200 amino acids and comprises at least the C-terminal six, preferably seven cysteine skeleton defined by residues 335-43-1 of Seq. ID No. 1, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra- or inter-chain disulfide bonds such that the protein is capable of acting as a morphogen as defined herein. The solubility of these structures is improved when the mature dimeric form of a morphogen, in accordance with the invention, is complexed with at least one, and preferably two, pro domains.

Various generic sequences (Generic Sequence 1-6) defining preferred C-terminal sequences useful in the soluble morphogens of this invention are described in WO 93/05751. Two currently preferred generic sequences are described below.

Generic Sequence 7 (Seq. ID No. 20) and Generic Sequence 8 (Seq. ID No. 21) disclosed below, accommodate the homologies shared among preferred morphogen protein family members identified to date, including OP-1, OP-2, OP-3, CBMP2A, CBMP2B, BMP3, 60A, DPP, Vgl, BMP5, BMP6, Vrg-1, and GDF-1. The amino acid sequences for these proteins are described herein (see Sequence Listing) and/or in the art, as well as in PCT publication US 92/07358 (WO93/04692) and US 91/07635 (WO92/07073), for example. The generic sequences include both the amino acid identity shared by these sequences in the C-terminal domain, defined by the six and seven cysteine skeletons (Generic Sequences 7 and 8, respectively), as well as alternative residues for the variable positions within the sequence. The generic sequences allow for an additional cysteine at position 41 (Generic Sequence 7) or position 46 (Generic Sequence 8), providing an appropriate cysteine skeleton where inter- or intramolecular disulfide bonds can form, and containing certain critical amino acids which influence the tertiary structure of the proteins. wherein each Xaa is independently selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp or Glu); Xaa at res.8 = (Leu, Val or Ile); Xaa at res.11 = (Gln, Leu, Asp, His, Asn or Ser); Xaa at res.12 = (Asp, Arg, Asn or Glu); Xaa at res. 13 = (Trp or Ser); Xaa at res.14 = (Ile or Val); Xaa at res.15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln, Ala or Ser); Xaa at res.28 = (Glu, Lys, Asp, Gln or Ala); Xaa at res.30 = (Ala, Ser, Pro, Gln, Ile or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu, Met or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val, Gly or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val, Pro or Lys); Xaa at res.56 = (Thr, Ala, Val, Lys, Asp, Tyr, Ser, Gly, Ile or His); Xaa at res.57 = (Val, Ala or Ile); Xaa at res.58 = (Pro or Asp); Xaa at res.59 = (Lys, Leu or Glu); Xaa at res.60 = (Pro, Val or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Leu, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn, Arg or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (Ile, Val or Asn); Xaa at res.84 = (Lys or Arg); Xaa at res.85 = (Lys, Asn, Gln, His, Arg or Val); Xaa at res.86 = (Tyr, Glu or His); Xaa at res.87 = (Arg, Gln, Glu or Pro); Xaa at res.88 = (Asn, Glu, Trp or Asp); Xaa at res.90 = (Val, Thr, Ala or Ile); Xaa at res.92 = (Arg, Lys, Val, Asp, Gln or Glu); Xaa at res.93 = (Ala, Gly, Glu or Ser); Xaa at res.95 = (Gly or Ala) and Xaa at res.97 = (His or Arg).

As described above, Generic Sequence 8 (Seq. ID No. 21) includes all of Generic Sequence 7 and in addition includes the following sequence at its N-terminus:

Accordingly, beginning with residue 7, each "Xaa" in Generic Seq. 8 is a specified amino acid defined as for Generic Seq. 7, with the distinction that each residue number described for Generic Sequence 7 is shifted by five in Generic Seq. 8. Thus, "Xaa at res.2 =(Tyr or Lys)" in Gen. Seq. 7 refers to Xaa at res. 7 in Generic Seq. 8. In Generic Seq. 8, Xaa at res.2 = (Lys, Arg, Ala or Gln); Xaa at res.3 = (Lys, Arg or Met); Xaa at res.4 = (His, Arg or Gln); and Xaa at res.5 = (Glu, Ser, His, Gly, Arg, Pro, Thr, or Tyr).

Accordingly, other useful sequences defining preferred C-terminal sequences are those sharing at least 70% amino acid sequence homology or "similarity", and preferably 80% homology or similarity with any of the sequences incorporated into Generic Seq. 7 and 8 above. These are anticipated to include allelic, species, chimeric and other sequence variants, (e.g., including "muteins" or "mutant proteins"), whether naturally-occurring or biosynthetically produced, as well as novel members of this morphogenic family of proteins. As used herein, "amino acid sequence homology" is understood to mean amino acid sequence similarity, and homologous sequences share identical or similar amino acids, where similar amino acids are conserved amino acids as defined by Dayoff et al., Atlas of Protein Sequence and Structure; vol.5, Suppl.3, pp.345-362 (M.O. Dayoff, ed., Nat'1 BioMed. Research Fdn., Washington D.C. 1978.) Thus, a candidate sequence sharing 70% amino acid homology with a reference sequence requires that, following alignment of the candidate sequence with the reference sequence, 70% of the amino acids in the candidate sequence are identical to the corresponding amino acid in the reference sequence, or constitute a conserved amino acid change thereto. "Amino acid sequence identity" is understood to require identical amino acids between two aligned sequences. Thus, a candidate sequence sharing 60% amino acid identity with a reference sequence requires that, following alignment of the candidate sequence with the reference sequence, 60% of the amino acids in the candidate sequence are identical to the corresponding amino acid in the reference sequence.

As used herein, all homologies and identities calculated use OP-1 as the reference sequence. Also as used herein, sequences are aligned for homology and identity calculations using the method of Needleman et al. (1970) J.Mol. Biol. 48:443-453 and identities calculated by the Align program (DNAstar, Inc.) In all cases, internal gaps and amino acid insertions in the candidate sequence as aligned are ignored when making the homology/identity calculation.

Also as used herein, "sequence variant" is understood to mean an amino acid sequence variant form of the morphogen protein, wherein the amino acid change or changes in the sequence do not alter significantly the morphogenic activity (e.g., tissue regeneration activity) of the protein, and the variant molecule performs substantially the same function in substantially the same way as the naturally-occurring form of the molecule. Sequence variants may include single or multiple amino acid changes, and are intended to include chimeric sequences as described below. The variants may be naturally-occurring or may be biosynthetically induced by using standard recombinant DNA techniques or chemical protein synthesis methodologies.

The currently most preferred protein sequences useful in soluble morphogen complexes in this invention include those having greater than 60% identity, preferably greater than 65% identity, with the amino acid sequence defining the conserved six cysteine skeleton of hOP1 (e.g., residues 335-431 of Seq. ID No. 1). These most preferred sequences include both allelic and species variants of the OP-1 and OP-2 proteins, including the Drosophila 60A protein. Accordingly, in another preferred aspect of the invention, useful morphogens include active proteins comprising species of polypeptide chains having the generic amino acid sequence herein referred to as "OPX", which accommodates the homologies between the various identified species of OP1 and OP2 (Seq. ID No. 22).

In still another preferred aspect of the invention, useful morphogens include active proteins comprising amino acid sequences encoded by nucleic acids that hydridize to DNA or RNA sequences encoding the conserved C-terminal cysteine domain of OP1 or OP2, e.g., defined by nucleotides 1036-1341 and nucleotides 1390-1695 of Seq. ID Nos. 1 and 5, respectively, under stringent hybridization conditions. As used herein, stringent hybridization conditions are defined as hybridization in 40% formamide, 5 X SSPE, 5 X Denhardt's Solution, and 0.1% SDS at 37°C overnight, and washing in 0.1 X SSPE, 0.1% SDS at 50°C. Similarly, in another preferred aspect of the invention, useful pro region peptides include polypeptide chains comprising amino acid sequences encoded by nucleic acids that hybridize to DNA or RNA sequences encoding at least the N-terminal 18 amino acids of the pro region sequences for any of the sequences listed in Seq. ID Nos. 1-19, under stringent hybridization conditions. Most preferably, the peptides are encoded by nucleic acids that hybridize to the DNA or RNA sequences encoding at least the N-terminal 18 amino acids of the pro region sequences for OP1 or OP2, e.g., nucleotides 136-192 and nucleotides 152-211 of Seq. ID Nos. 1 and 5, respectively.

Useful N-terminal extension sequences are listed in Fig. 2 for use with the C-terminal domains described above. Also as described above, the full length N-terminal extensions, or truncated forms thereof, may be used in preferred dimeric species. The mature dimeric species may be produced from intact DNAs, or truncated forms thereof. It also is envisioned as an embodiment of the invention that chimeric morphogen sequences can be used. Thus, DNAs encoding chimeric morphogens may be constructed using part or all of the N-terminal extension from one morphogen and a C-terminal domain derived from one or more other morphogens. These chimeric proteins may be synthesized using standard recombinant DNA methodology and/or automated chemical nucleic acid synthesis methodology well described in the art. Other chimeric morphogens include soluble morphogen complexes where the pro domain is encoded from a DNA sequence corresponding to one or more morphogen pro sequences, and part or all of the mature domain is encoded by DNA derived from one or more other, different morphogens. These soluble chimerics may be produced from a single synthetic DNA as described below, or, alternatively, may be formulated in vitro from isolated components also as described herein below.

Finally, the morphogen pro domains and/or mature form N-terminal extensions themselves may be useful as tissue targeting sequences. As described above, the morphogen family members share significant sequence homology in their C-terminal active domains. By contrast, the sequences diverge significantly in the sequences which define the pro domain and the N-terminal 39 amino acids of the mature protein. Accordingly, the pro domain and/or N-terminal extension sequence may be morphogen-specific. Accordingly, part or all of these morphogen-specific sequences may serve as tissue targeting sequences for the morphogens described herein. For example, the N-terminal extension and/or pro domains may interact specifically with one or more molecules at the target tissue to direct the morphogen associated with the pro domain to that tissue. Thus, for example, the morphogen-specific sequences of OP-1, BMP2 or BMP4, all of which proteins are found naturally associated with bone tissue (see, for example, US Pat. No. 5,011,691) may be particularly useful sequences when the morphogen complex is to be targeted to bone. Similarly, BMP6 (or Vgr-1) specific sequences may be used when targeting to lung tissue is desired. Alternatively, the morphogen-specific sequences of GDF-1 may be used to target soluble morphogen complexes to nerve tissue, particularly brain tissue, where GDF-1 appears to be primarily expressed (see, for example, Lee, PNAS, 88:4250-4254 (1991), incorporated herein by reference).

### II. Recombinant Production of Soluble Morphogen Complexes

Soluble morphogen complexes can be produced from eukaryotic host cells, preferably mammalian cells, using standard recombinant expression techniques. An exemplary protocol currently preferred, is provided below, using a particular vector construct and chinese hamster ovary (CHO) cell line. Those skilled in the art will appreciate that other expression systems are contemplated to be useful, including other vectors and other cell systems, and the invention is not intended to be limited to soluble morphogenic protein complexes produced only by the method detailed hereinbelow. Similar results to those described herein have been observed using recombinant expression systems developed for COS and BSC cells.

Morphogen DNA encoding the precursor sequence is subcloned into an insertion site of a suitable, commercially available pUC-type vector (e.g., pUC-19, ATCC #37254, Rockville, MD), along with a suitable promoter/enhancer sequences and 3' termination sequences. Useful DNA sequences include the published sequences encoding these proteins, and/or synthetic constructs. Currently preferred promoter/enhancer sequences are the CMV promoter (human cytomegalovirus major intermediate - early promoter) and the mouse mammary tumor virus promoter (mMTV) boosted by the rous sarcoma virus LTR enhancer sequence (e.g., from Clontech, Inc., Palo Alto). Expression also may be further enhanced using transactivating enhancer sequences. The plasmid also contains DHFR as an amplifiable marker, under SV40 early promoter control (ATCC #37148). Transfection, cell culturing, gene amplification and protein expression conditions are standard conditions, well known in the art, such as are described, for example in Ausubel et al., ed., Current Protocols in Molecular Biology, John Wiley & Sons, NY (1989). Briefly, transfected cells are cultured in medium containing 0.1-0.5% dialyzed fetal calf serum (FCS) and stably transfected high expression cell lines are obtained by subcloning and evaluated by standard Western or Northern blot. Southern blots also are used to assess the state of integrated sequences and the extent of their copy number amplification.

A currently preferred expression vector contains the DHFR gene, under SV40 early promoter control, as both a selection marker and as an inducible gene amplifier. The DNA sequence for DHFR is well characterized in the art, and is available commercially. For example, a suitable vector may be generated from pMAM-neo (Clontech, Inc., Palo Alto, CA) by replacing the neo gene (BamHI digest) with an SphI-BamHI, or a PvuII-BamHI fragment from pSV5-DHFR (ATCC #37148), which contains the DHFR gene under SV40 early promoter control. A BamHI site can be engineered at the SphI or PvuII site using standard techniques (e.g., by linker insertion or site-directed mutagenesis) to allow insertion of the fragment into the vector backbone. The morphogen DNA can be inserted into the polylinker site downstream of the MMTV-LTR sequence (mouse mammary tumor virus LTR). The CMV promoter sequence then may be inserted into the expression vector (e.g., from pCDM8, Invitrogen, Inc.) The SV40 early promoter, which drives DHFR expression, preferably is modified in these vectors to reduce the level of DHFR mRNA produced.

The currently preferred mammalian cell line is a CHO Chinese hamster ovary, cell line, and the preferred procedure for establishing a stable morphogen production cell line with high expression levels comprises transfecting a stable CHO cell line, preferably CHO-DXB11, with the expression vector described above, isolating clones with high snorphogen expression levels, and subjecting these clones to cycles of subcloning using a limited dilution method described below to obtain a population of high expression clones. Subcloning preferably is performed in the absence of MTX to identify stable high expression clones which do not require addition of MTX to the growth media for morphogen production.

In the subcloning protocol cells are seeded on ten 100mm petri dishes at a cell density of either 50 or 100 cells per plate, with or preferably without MTX in the culture media. After 14 days of growth, clones are isolated using cloning cylinders and standard procedures, and cultured in 24-well plates. Clones then are screened for morphogen expression by Western immunoblots using standard procedures, and morphogen expression levels compared to parental lines. Cell line stability of high expression subclones then is determined by monitoring morphogen expression levels over multiple cell passages (e.g., four or five passages).

### III. Isolation of Soluble morphogen complex from conditioned media or body fluid

Morphogens are expressed from mammalian cells as soluble complexes. Typically, however the complex is disassociated during purification, generally by exposure to denaturants often added to the purification solutions, such as detergents, alcohols, organic solvents, chaotropic agents and compounds added to reduce the pH of the solution. Provided below is a currently preferred protocol for purifying the soluble proteins from conditioned media (or, optionally, a body fluid such as serum, cerebro-spinal or peritoneal fluid), under non-denaturing conditions. The method is rapid, reproducible and yields isolated soluble morphogen complexes in substantially pure form.

Soluble morphogen complexes can be isolated from conditioned media using a simple, three step chromatographic protocol performed in the absence of denaturants. The protocol involves running the media (or body fluid) over an affinity column, followed by ion exchange and gel filtration chromatographies. The affinity column described below is a Zn-IMAC column. The present protocol has general applicability to the purification of a variety of morphogens, all of which are anticipated to be isolatable using only minor modifications of the protocol described below. An alternative protocol also envisioned to have utility an immunoaffinity column, created using standard procedures and, for example, using antibody specific for a given morphogen pro domain (complexed, for example, to a protein A-conjugated Sepharose column.) Protocols for developing immunoaffinity columns are well described in the art, (see, for example, Guide to Protein Purification, M. Deutscher, ed., Academic Press, San Diego, 1990, particularly sections VII and XI.)

In this experiment OP-1 was expressed in CHO cells as described above. The CHO cell conditioned media containing 0.5% FBS was initially purified using Immobilized Metal-Ion Affinity Chromatography (IMAC). The soluble OP-1 complex from conditioned media binds very selectively to the Zn-IMAC resin and a high concentration of imidazole (50 mM imidazole, pH 8.0) is required for the effective elution of the bound complex. The Zn-IMAC step separates the soluble OP-1 from the bulk of the contaminating serum proteins that elute in the flow through and 35 mM imidazole wash fractions. The Zn-IMAC purified soluble OP-1 is next applied to an S-Sepharose cation-exchange column equilibrated in 20 mM NaPO₄ (pH 7.0) with 50 mM NaCl. This S-Sepharose step serves to further purify and concentrate the soluble OP-1 complex in preparation for the following gel filtration step. The protein was applied to a Sephacryl S-200HR column equilibrated in TBS. Using substantially the same protocol, soluble morphogens also may be isolated from one or more body fluids, including serum, cerebro-spinal fluid or peritoneal fluid.

IMAC was performed using Chelating-Sepharose (Pharmacia) that had been charged with three column volumes of 0.2 M ZnSO₄. The conditioned media was titrated to pH 7.0 and applied directly to the ZN-IMAC resin equilibrated in 20 mM HEPES (pH 7.0) with 500 mM NaCl. The Zn-IMAC resin was loaded with 80 mL of starting conditioned media per mL of resin. After loading the column was washed with equilibration buffer and most of the contaminating proteins were eluted with 35 mM imidazole (pH 7.0) in equilibration buffer. The soluble OP-1 complex is then eluted with 50 mM imidazole (pH 8.0) in 20 mM HEPES and 500 mM NaCl.

The 50 mM imidazole eluate containing the soluble OP-1 complex was diluted with nine volumes of 20 mM NaPO₄ (pH 7.0) and applied to an S-Sepharose (Pharmacia) column equilibrated in 20 mM NaPO₄ (pH 7.0) with 50 mM NaCl. The S-Sepharose resin was loaded with an equivalent of 800 mL of starting conditioned media per mL of resin. After loading the S-Sepharose column was washed with equilibration buffer and eluted with 100 mM NaCl followed by 300 mM and 500 mM NaCl in 20 mM NaPO₄ (pH 7.0). The 300 mM NaCl pool was further purified using gel filtration chromatography. Fifty mls of the 300 mm NaCl eluate was applied to a 5.0 X 90 cm Sephacryl S-200HR (Pharmacia) equilibrated in Tris buffered saline (TBS), 50 mM Tris, 150 mM NaCl (pH 7.4). The column was eluted at a flow rate of 5 mL/minute collecting 10 mL fractions. The apparent molecular of the soluble OP-1 was determined by comparison to protein molecular weight standards (alcohol dehydrogenase (ADH, 150 kDa), bovine serum albumin (BSA, 68 kDa), carbonic anhydrase (CA, 30 kDa) and cytochrome C (cyt C, 12.5 kDa). (see Fig. 3) The purity of the S-200 column fractions was determined by separation on standard 15% polyacrylamide SDS gels stained with coomassie blue. The identity of the mature OP-1 and the pro-domain was determined by N-terminal sequence analysis after separation of the mature OP-1 from the pro-domain using standard reverse phase C18 HPLC.

Figure 3 shows the absorbance profile at 280 nm. The soluble OP-1 complex elutes with an apparent molecular weight of 110 kDa. This agrees well with the predicted composition of the soluble OP-1 complex with one mature OP-1 dimer (35-36 kDa) associated with two pro-domains (39 kDa each). Purity of the final complex can be verified by running the appropriate fraction in a reduced 15% polyacrylamide gel.

The complex components can be verified by running the complex-containing fraction from the S-200 or S-200HR columns over a reverse phase C18 HPLC column and eluting in an acetonitrile gradient (in 0.1% TFA), using standard procedures. The complex is dissociated by this step, and the pro domain and mature species elute as separate species. These separate species then can be subjected to N-terminal sequencing using standard procedures (see, for example, Guide to Protein Purification, M. Deutscher, ed., Academic Press, San Diego, 1990, particularly pp. 602-613), and the identity of the isolated 36kD, 39kDa proteins confirmed as mature morphogen and isolated, cleaved pro domain, respectively. N-terminal sequencing of the isolated pro domain from mammalian cell produced OP-1 revealed 2 forms of the pro region, the intact form (beginning at residue 30 of Seq. ID No. 1) and a truncated form, (beginning at residue 48 of Seq. ID No. 1.) N-terminal sequencing of the polypeptide subunit of the isolated mature species reveals a range of N-termini for the mature sequence, beginning at residues 293, 300, 313, 315, 316, and 318, of Seq. ID No. 1, all of which are active as demonstrated by the standard bone induction assay.

### V. In Vitro Soluble Morphogen Complex Formation

As an alternative to purifying soluble complexes from culture media or a body fluid, soluble complexes may be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain has an opportunity to associate with the mature dimeric species under relaxed folding conditions. The concentration of denaturant in the solution then is decreased in a controlled, preferably step-wise manner, so as to allow proper refolding of the dimer and pro regions while maintaining the association of the pro domain with the dimer. Useful denaturants include 4-6M urea or guanidine hydrochloride (GuHCl), in buffered solutions of pH 4-10, preferably pH 6-8. The soluble complex then is formed by controlled dialysis or dilution into a solution having a final denaturant concentration of less than 0.1-2M urea or GuHCl, preferably 1-2 M urea of GuHCl, which then preferably can be diluted into a physiological buffer. Protein purification/renaturing procedures and considerations are well described in the art, and details for developing a suitable renaturing protocol readily can be determined by one having ordinary skill in the art. One useful text one the subject is Guide to Protein Purification, M. Deutscher, ed., Academic Press, San Diego, 1990, particularly section V. Complex formation also may be aided by addition of one or more chaperone proteins.

### VI. Stability of Soluble Morphogen Complexes

The stability of the highly purified soluble morphogen complex in a physiological buffer, e.g., tris-buffered saline (TBS) and phosphate-buffered saline (PBS), can be enhanced by any of a number of means. Currently preferred is by means of a pro region that comprises at least the first 18 amino acids of the pro sequence (e.g., residues 30-47 of Seq. ID NO. 1 for OP-1), and preferably is the full length pro region. Residues 30-47 show sequence homology to the N-terminal portion of other morphogens and are believed to have particular utility in enhancing complex stability for all morphogens. Other useful means for enhancing the stability of soluble morphogen complexes include three classes of additives. These additives include basic amino acids (e.g., L-arginine, lysine and betaine); nonionic detergents (e.g., Tween 80 or NonIdet P-120); and carrier proteins (e.g., serum albumin and casein). Useful concentrations of these additives include 1-100 mM, preferably 10-70 mM, including 50 mM, basic amino acid;, 0.01-1.0%, preferably 0.05-0.2%, including 0.1% (v/v) nonionic detergent;, and 0.01-1.0%, preferably 0.05-0.2%, including 0.1% (w/v) carrier protein.

### VII. Activity of Soluble Morphogen Complex

Association of the pro domain with the mature dimeric species does not interfere with the morphogenic activity of the protein in vivo as demonstrated by different activity assays. Specifically, soluble OP-1 complex provided in a standard rat osteopenia model induces significant increase in bone growth and osteocalcin production (see Table II, below), in a manner analogous to the results obtained using mature morphogen.

The assay is analogous to the osteoporosis model described in international application US92/07432 (WO93/05751), but uses aged female rats rather than ovariectomized animals. Briefly, young or aged female rats (Charles River Labs, 115-145, and 335-460g body weight, respectively) were dosed daily for 7 days by intravenous tail injection, with either 20 µg/Kg body weight soluble OP-1, or 100 µg/Kg body weight soluble OP-1. Control groups of young and aged female rats were dosed only with tris-buffered saline (TBS). Water and food were provided to all animals ad libitum. After 14 days, animals were sacrificed, and new bone growth measured by standard histometric procedures. Osteocalcin concentrations in serum also were measured. No detrimental effects of morphogen administration were detected as determined by changes in animal body or organ weight or by hematology profiles.

**TABLE II**

| No. Animals | Animal Group | Bone Area (B.Ar/T.Ar) | Osteocalcin (ng/ml) |
|---|---|---|---|
| 4 | Control | 5.50 ± 0.64 | 11.89 ± 4.20 |
| 5 | Aged female, 20µg/Kg sol. OP-1 | 7.68 ± 0.63** | 22.24 ± 2.28** |
| 5 | Aged female, 100µg/Kg sol. OP-1 | 9.82 ± 3.31* | 20.87 ± 6.14* |

| | | | |
|---|---|---|---|
| *P < 0.05 | | | |
| **P < 0.01 | | | |

Similar experiments performed using soluble OP-1 complex in the osteoporosis model described in WO93/05751 using ovariectomized rats also show no detrimental effect using the complex form.

Both mature and soluble morphogen also can induce CAM (cell adhesion molecule) expression, as demonstrated below. Briefly, induction of N-CAM isoforms (N-CAM-180, N-CAM-140 and N-CAM-120) can be monitored by reaction with the commercially available antibody mAb H28.123 (Sigma Co., St. Louis) and available antibody mAb H28.123 (Sigma Co., St. Louis) and standard Western blot analysis (see, for example, Molecular Cloning, A Laboratory Manual, Sambrook et al. eds. Cold Spring Harbor Press, New York, 1989, particularly Section 18). Incubation of a growing culture of transformed cells of neuronal origin, NG108-15 cels (ATCC, Rockville, MD) with either mature morphogen dimers or soluble morphogen complexes (10-100 ng/ml, preferably at least 40 ng/ml) induces a redifferentiation of these cells back to a morphology characteristic of untransformed neurons, including specific induction and/or enhanced expression of all 3 N-CAM isoforms. In the experiment, cells were subcultured on poly-L-lysine coated 6-well plates and grown in chemically defined medium for 2 days before the experiment. Fresh aliquots of morphogen were added (2.5µl) daily.

### VIII. Antibody Production

Provided below are standard protocols for polycolonal and monoclonal antibody production. For antibodies which recognize the soluble complex only, preferably the isolated pro region is used as the antigen; where antibodies specific to the mature protein are desired, the antigen preferably comprises at least the C-terminal domain or the intact mature sequence.

Polyclonal antibody may be prepared as follows. Each rabbit is given a primary immunization of 100 ug/500 µl of antigen, in 0.1% SDS mixed with 500 µl Complete Freund's Adjuvant. The antigen is injected subcutaneously at multiple sites on the back and flanks of the animal. The rabbit is boosted after a month in the same manner using incomplete Freund's Adjuvant. Test bleeds are taken from the ear vein seven days later. Two additional boosts and test bleeds are performed at monthly intervals until antibody against the morphogen antigen is detected in the serum using an ELISA assay. Then, the rabbit is boosted monthly with 100 µg of antigen and bled (15 ml per bleed) at days seven and ten after boosting.

Monoclonal antibody specific for a given morphogen may be prepared as follows. A mouse is given two injections of the morphogen antigen. The protein or protein fragment preferably is recombinantly produced. The first injection contains 100µg of antigen in complete Freund's adjuvant and is given subcutaneously. The second injection contains 50 µg of antigen in incomplete adjuvant and is given intraperitoneally. The mouse then receives a total of 230 µg of OP-3 in four intraperitoneal injections at various times over an eight month period. One week prior to fusion, the mouse is boosted intraperitoneally with antigen (e.g., 100 µg) and may be additionally boosted with a peptide fragment conjugated to bovine serum albumin with a suitable crosslinking agent. This boost can be repeated five days (IP), four days (IP), three days (IP) and one day (IV) prior to fusion. The mouse spleen cells then are fused to commercially available myeloma cells at a ratio of 1:1 using PEG 1500 (Boeringer Mannheim, Germany), and the fused cells plated and screened for mature or soluble morphogen-specific antibodies using the appropriate portion of the morphogen sequence as antigen. The cell fusion and monoclonal screening steps readily are performed according to standard procedures well described in standard texts widely available in the art.

Using these standard procedures, anti-pro domain antisera was prepared from rabbits using the isolated pro domain from OP-1 as the antigen, and monoclonal antibody ("mAb") to the mature domain was produced in mice, using an E. coli-produced truncated form of OP-1 as antigen.

Standard Western blot analysis performed under reducing conditions demonstrates that the anti-pro domain antisera ("anti-pro") is specific for the pro domain only, while the mAb to mature OP-1 ("anti-mature OP-1") is specific for the dimer subunits, that the two antibodies do not cross-react, and that the antibodies and can be used to distinguish between soluble and mature protein forms in a sample, e.g., of conditioned media or serum. A tabular representation of the Western blot results is in Table III below, where reactivity of mAb to mature OP-1 is indicated by "yy", and reactivity of the anti-pro antisera is indicated by "xx".

**TABLE III**

| Antibody | Purified Sol OP1 | Conditioned CHO Cell Media | Isolated Pro Domain | Purified Dimer Subunits |
|---|---|---|---|---|
| "anti-pro" | xx | xx | xx | |
| "anti-mature OP-1" | yy | yy | | yy |

### IX. Immunoassays

The ability to detect morphogens in solution and to distinguish between soluble and mature dimeric morphogen forms provides a valuable tool for diagnostic assays, allowing one to monitor the level and type of morphogen free in the body, e.g., in serum and other body fluids, as well as to develop diagnostic and other tissue evaluating kits.

For example, OP-1 is an intimate participant in normal bone growth and resorption. Thus, soluble OP-1 is expected to be detected at higher concentrations in individuals experiencing high bone turnover, such as children, and at substantially lower levels in individuals with abnormally low rates of bone turnover, such as patients with osteoporosis, osteosarcoma, Paget's disease and the like. Monitoring the level of OP-1, or other bone targeted morphogens such as BMP2 and BMP4, in serum thus provides a means for evaluating the status of bone tissue in an individual, as well as a means for monitoring the efficacy of a treatment to regenerate damaged or lost bone tissue. Similarly, monitoring the level of endogenous GDF-1, can provide diagnostic information on the health of nerve tissue, particularly brain tissue. Moreover, following this disclosure one can distinguish between the level of soluble and mature forms in solution.

A currently preferred detection means for evaluating the level of morphogen in a body fluid comprises an immunoassay utilizing an antibody or other suitable binding protein capable of reacting specifically with a morphogen and being detected as part of a complex with the morphogen. Immunoassays may be performed using standard techniques known in the art and antibodies raised against a morphogen and specific for that morphogen. Antibodies which recognize a morphogen protein form of interest may be generated as described herein and these antibodies then used to monitor endogenous levels of protein in a body fluid, such as serum, whole blood or peritoneal fluid. To monitor endogenous concentrations of soluble morphogen, the antibody chosen preferably has binding specificity for the soluble form e.g., has specificity for the pro domain. Such antibodies may be generated by using the pro domain or a portion thereof as the antigen, essentially as described herein. A suitable pro domain for use as an antigen may be obtained by isolating the soluble complex and then separating the noncovalently associated pro domain from the mature domain using standard procedures, e.g., by passing the complex over an HPLC column, as described above or by separation by gel electrophoresis. Alternatively, the pro form of the protein in its monomeric form may be used as the antigen and the candidate antibodies screened by Western blot or other standard immunoassay for those which recognize the pro domain of the soluble form of the protein of interest, but not the mature form, also as described above.

Monomeric pro forms can be obtained from cell lysates of CHO produced cells, or from prokaryotic expression of a DNA encoding the pro form, in for example, E.coli. The pro form, which has an apparent molecular weight of about 50 kDa in mammalian cells, can then be isolated by HPLC and/or by gel electrophoresis, as described above.

In order to detect and/or quantitate the amount of morphogenic protein present in a solution, an immunoassay may be performed to detect the morphogen using a polyclonal or monoclonal antibody specific for that protein. Here, soluble and mature forms of the morphogen also may be distinguished by using antibodies that discriminate between the two forms of the proteins as described above. Currently preferred assays include ELISAS and radioimmunassays, including standard competitor assays useful for quantitating the morphogen in a sample, where an unknown amount of sample morphogen is allowed to react with anti-morphogen antibody and this interaction is competed with a known amount of labeled antigen. The level of bound or free labeled antigen at equilibrium then is measured to quantitate the amount of unlabeled antigen in solution, the amount of sample antigen being proportional to the amount of free labeled antigen. Exemplary protocols for these assays are provided below. However, as will be appreciated by those skilled in the art, variations of these protocols, as well as other immunoassays, are well known in the literature and within the skill of the art. For example, in the ELISA protocol provided below, soluble OP-1 is identified in a sample using biotinylated anti-pro antiserum. Biotinylated antibodies can be visualized in a colormetric assay or in a chemiluminescent assay, as described below. Alternatively, the antibody can be radio-labeled with a suitable molecule, such as ¹²⁵I. Still another protocol that may be used is a solid phase immunoassay, preferably using an affinity column with anti-morphogen antibody complexed to the matrix surface and over which a serum sample may be passed. A detailed description of useful immunoassays, including protocols and general considerations is provided in, for example, Molecular Cloning: A Laboratory Manual, Sambrook et al., eds. Cold Spring Harbor Press, New York, 1989, particularly Section 18.

For serum assays, the serum preferably first is partially purified to remove some of the excess, contaminating serum proteins, such as serum albumin. Preferably the serum is extracted by precipitation in ammonium sulfate (e.g., 45%) such that the complex is precipitated. Further purification can be achieved using purification strategies that take advantage of the differential solubility of soluble morphogen complex or mature morphogens relative to that of the other proteins present in serum. Further purification also can be achieved by chromatographic techniques well known in the art.

Soluble OP-1 may be detected using a polyclonal antibody specific for the OP-1 pro domain in an ELISA, as follows. 1 µg/100 µl of affinity-purified polyclonal rabbit IgG specific for OP-1-pro is added to each well of a 96-well plate and incubated at 37°C for an hour. The wells are washed four times with 0.167M sodium borate buffer with 0.15 M NaCl (BSB), pH 8.2, containing 0.1% Tween 20. To minimize non-specific binding, the wells are blocked by filling completely with 1% bovine serum albumin (BSA) in BSB and incubating for 1 hour at 37°C. The wells are then washed four times with BSB containing 0.1% Tween 20. A 100 µl aliquot of an appropriate dilution of each of the test samples of cell culture supernatant or serum sample is added to each well in triplicate and incubated at 37°C for 30 min. After incubation, 100 µl biotinylated rabbit anti-pro serum (stock solution is about 1 mg/ml and diluted 1:400 in BSB containing 1% BSA before use) is added to each well and incubated at 37°C for 30 min. The wells are then washed four times with BSB containing 0.1% Tween 20. 100 µl strepavidin-alkaline (Southern Biotechnology Associates, Inc. Birmingham, Alabama, diluted 1:2000 in BSB containing 0.1% Tween 20 before use) is added to each well and incubated at 37°C for 30 min. The plates are washed four times with 0.5M Tris buffered Saline (TBS), pH 7.2. 50µl substrate (ELISA Amplification System Kit, Life Technologies, Inc., Bethesda, MD) is added to each well incubated at room temperature for 15 min. Then, 50 µl amplifier (from the same amplification system kit) is added and incubated for another 15 min at room temperature. The reaction is stopped by the addition of 50 µl 0.3 M sulphuric acid. The OD at 490 nm of the solution in each well is recorded. To quantitate the level of soluble OP-1 in the sample, a standard curve is performed in parallel with the test samples. In the standard curve, known increasing amounts of purified OP-1-pro is added. Alternatively, using, for example, Lumi-phos 530 (Analytical Luminescence Laboratories) as the substrate and detection at 300-650 nm in a standard luminometer, complexes can be detected by chemiluminescence, which typically provides a more sensitive assay than detection by means of a visible color change.

Morphogen (soluble or mature form) may be detected in a standard plated-based radioimmunoassay as follows. Empirically determined limiting levels of anti-morphogen antibody (e.g., anti-OP-1, typically 50-80 ng/well) are bound to wells of a PVC plate e.g., in 50 µl PBS phosphate buffered saline. After sufficient incubation to allow binding at room temperature, typically one hour, the plate is washed in a PBS/Tween 20 solution, ("washing buffer"), and 200 µl of block (3% BSA, 0.1µ lysine in 1xBSB) is added to each well and allowed to incubate for 1 hour, after which the wells are washed again in washing buffer. 40 µl of a sample composed of serially diluted plasma (preferably partially purified as described above) or morphogen standard (e.g., OP-1) is added to wells in triplicate. Samples preferably are diluted in PTTH (15 mM KH₂PO₄, 8 mM Na₂PO₄, 27 mM KCl, 137 mM NaCl, 0.05% Tween 20, 1 mg/ml HSA, 0.05% NaN₃, pH 7.2). 10 µl of labelled competitor antigen, preferably 100,000-500,000 cpm/sample is added (e.g., ¹²⁵I OP-1, radiolabelled using standard procedures), and plates are incubated overnight at 4°C. Plates then are washed in washing buffer, and allowed to dry. Wells are cut apart and bound labelled OP-1 counted in a standard gamma counter. The quantities of bound labelled antigen (e.g., ¹²⁵I OP-1) measured in the presence and absence of sample then are compared, the difference being proportional to the amount of sample antigen (morphogen) present in the sample fluid.

As a corollary assay method, immunoassays may be developed to detect endogenous anti-morphogen antibodies, and to distinguish between such antibodies to soluble or mature forms. Endogenous anti-morphogen antibodies have been detected in serum, and their level is known to increase, for example, upon implanting of an osteogenic device in a mammal. Without being limited to a particular theory, these antibodies may play a role in modulating morphogen activity by modulating the level of available protein in serum. Assays that monitor the level of endogenous antibodies in blood or their body fluids thus can be used in diagnostic assays to evaluate the status of a tissue, as well as to provide a means for monitoring the efficacy of a therapy for tissue regeneration.

The currently preferred means for detecting endogenous anti-morphogen antibodies is by means of a standard Western blot. See, for example, Molecular Cloning: A Laboratory Manual Sambrook et al., eds., Cold Spring Harbor Press, New York, 1989, particularly pages 18.60-18.75, incorporated herein by reference, for a detailed description of these assays. Purified mature or soluble morphogen is electrophoresed on an SDS polyacrylamide gel under oxidized or reduced conditions designed to separate the proteins in solution, and the proteins then transferred to a polyvinylidene difluoride microporus membrane (0.45 µm pore sizes) using standard buffers and procedures. The filter then is incubated with the serum being tested (at various dilutions). Antibodies bound to either the pro domain or the mature morphogen domain are detected by means of an anti-human antibody protein, e.g., goat anti-human Ig. Titers of the antimorphogen antibodies can be determined by further dilution of the serum until no signal is detected.

### X. Formulations and Methods for Administering Soluble Morphogens as Therapeutic Agents

The soluble morphogens of this invention are particularly useful as therapeutic agents to regenerate diseased or damaged tissue in a mammal, particularly a human.

The soluble morphogen complexes may be used to particular advantage in regeneration of damaged or diseased lung, heart, liver, kidney, nerve or pancreas tissue, as well as in the transplantation and/or grafting of these tissues and bone marrow, skin, gastrointestinal mucosa, and other living tissues.

The soluble morphogen complexes described herein may be provided to an individual by any suitable means, preferably directly or systemically, e.g., parenterally or orally. Where the morphogen is to be provided directly (e.g., locally, as by injection, to a desired tissue site), or parenterally, such as by intravenous, subcutaneous, intramuscular, intraorbital, ophthalmic, intraventricular, intracranial, intracapsular, intraspinal, intracisternal, intraperitoneal, buccal, rectal, vaginal, intranasal or by aerosol administration, the soluble morphogen complex preferably comprises part of an aqueous solution. The solution is physiologically acceptable so that in addition to delivery of the desired morphogen to the patient, the solution does not otherwise adversely affect the patient's electrolyte and volume balance. The aqueous medium for the soluble morphogen thus may comprise normal physiologic saline (0.9% NaCl, 0.15M), pH 7-7.4.

Soluble morphogens of this invention are readily purified from cultured cell media into a physiological buffer, as described above. In addition, and as described above, if desired, the soluble complexes may be formulated with one or more additional additives, including basic amino acids (e.g., L-arginine, lysine, betaine); non-ionic detergents (e.g. Tween-80 or NonIdet-120) and carrier proteins (e.g., serum albumin and casein).

Useful solutions for oral or parenteral administration may be prepared by any of the methods well known in the pharmaceutical art, described, for example, in Remington's Pharmaceutical Sciences, (Gennaro, A., ed.), Mack Pub., 1990. Formulations may include, for example, polyalkylene glycols such as polyethylene glycol, oils of vegetable origin, hydrogenated naphthalenes, and the like. Formulations for direct administration, in particular, may include glycerol and other compositions of high viscosity. Biocompatible, preferably bioresorbable polymers, including, for example, hyaluronic acid, collagen, tricalcium phosphate, polybutyrate, polylactide, polyglycolide and lactide/glycolide copolymers, may be useful excipients to control the release of the soluble morphogen in vivo.

Other potentially useful parenteral delivery systems for these morphogens include ethylene-vinyl acetate copolymer particles, osmotic pumps, implantable infusion systems, and liposomes. Formulations for inhalation administration may contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally.

The soluble morphogens described herein also may be administered orally. Oral administration of proteins as therapeutics generally is not practiced as most proteins readily are degraded by digestive enzymes and acids in the mammalian digestive system before they can be absorbed into the bloodstream. However, the mature domains of the morphogens described herein typically are acid-stable and protease-resistant (see, for example, U.S. Pat. No. 4,968,590.) In addition, at least one morphogen, OP-1, has been identified, in mammary gland extract, colostrum and milk, as well as saliva. Moreover, the OP-1 purified from mammary gland extract is morphogenically active. For example, this protein induces endochondral bone formation in mammals when implanted subcutaneously in association with a suitable matrix material, using a standard in vivo bone assay, such as is disclosed in U.S. Pat. No. 4,968,590. In addition, endogenous morphogen also is detected in human serum (see above). Finally, comparative experiments with soluble and mature morphogens in a number of experiments defining morphogenic activity indicate that the non-covalent association of the pro domain with the dimeric species does not interfere with morphogenic activity. These findings indicate that oral and parenteral administration are viable means for administering morphogens to an individual, and that soluble morphogens have utility in systemic administration protocols.

The soluble complexes provided herein also may be associated with molecules capable of targeting the morphogen to a desired tissue. For example, tetracycline and diphosphonates (bisphosphonates) are known to bind to bone mineral, particularly at zones of bone remodeling, when they are provided systemically in a mammal. Accordingly, these molecules may be included as useful agents for targeting soluble morphogens to bone tissue. Alternatively, an antibody or other binding protein that interacts specifically with a surface molecule on the desired target tissue cells also may be used. Such targeting molecules further may be covalently associated to the morphogen complex, e.g., by chemical crosslinking, or by using standard genetic engineering means to create, for example, an acid labile bond such as an Asp-Pro linkage. Useful targeting molecules may be designed, for example, using the single chain binding site technology disclosed, for example, in U.S. Pat. No. 5,091,513. Finally, the soluble morphogen complexes provided herein may be administered alone or in combination with other molecules known to have a beneficial effect on tissue morphogenesis, including molecules capable of tissue repair and regeneration and/or inhibiting inflammation. Examples of useful cofactors for stimulating bone tissue growth in osteoporotic individuals, for example, include but are not limited to, vitamin D₃, calcitonin, prostaglandins, parathyroid hormone, dexamethasone, estrogen and IGF-I or IGF-II. Useful cofactors for nerve tissue repair and regeneration may include nerve growth factors. Other useful cofactors include symptom-alleviating cofactors, including antiseptics, antibiotics, antiviral and antifungal agents and analgesics and anesthetics.

The compounds provided herein can be formulated into pharmaceutical compositions by admixture with pharmaceutically acceptable nontoxic excipients and carriers. As noted above, such compositions may be prepared for parenteral administration, particularly in the form of liquid solutions or suspensions; for oral administration, particularly in the form of tablets or capsules; or intranasally, particularly in the form of powders, nasal drops or aerosols. Where adhesion to a tissue surface is desired the composition may include the morphogen dispersed in a fibrinogen-thrombin composition or other bioadhesive. The composition then may be painted, sprayed or otherwise applied to the desired tissue surface.

The compositions can be formulated for parenteral or oral administration to humans or other mammals in therapeutically effective amounts, e.g., amounts which provide appropriate concentrations of the morphogen to target tissue for a time sufficient to induce morphogenesis, including particular steps thereof, as described above.

Where the soluble morphogen complex is to be used as part of a transplant procedure, the morphogen may be provided to the living tissue or organ to be transplanted prior to removal of the tissue or organ from the donor. The morphogen may be provided to the donor host directly, as by injection of a formulation comprising the soluble complex into the tissue, or indirectly, e.g., by oral or parenteral administration, using any of the means described above.

Alternatively or, in addition, once removed from the donor, the organ or living tissue may be placed in a preservation solution containing the morphogen. In addition, the recipient also preferably is provided with the morphogen just prior to, or concommitant with, transplantation. In all cases, the soluble complex may be administered directly to the tissue at risk, as by injection to the tissue, or it may be provided systemically, either by oral or parenteral administration, using any of the methods and formulations described herein and/or known in the art.

Where the morphogen comprises part of a tissue or organ preservation solution, any commercially available preservation solution may be used to advantage. A useful preservation solution is described in in PCT/US92/07358 (WO93/04692).

As will be appreciated by those skilled in the art, the concentration of the compounds described in a therapeutic composition will vary depending upon a number of factors, including the dosage of the drug to be administered, the chemical characteristics (e.g., hydrophobicity) of the compounds employed, and the route of administration. The preferred dosage of drug to be administered also is likely to depend on such variables as the type and extent of tissue loss or defect, the overall health status of the particular patient, the relative biological efficacy of the compound selected, the formulation of the compound, the presence and types of excipients in the formulation, and the route of administration. In general terms, the compounds of this invention may be provided in an aqueous physiological buffer solution containing about 0.001 to 10% w/v compound for parenteral administration. Typical dose ranges are from about 10 ng/kg to about 1 g/kg of body weight per day; a preferred dose range is from about 0.1 µg/kg to 100 mg/kg of body weight. No obvious morphogen-induced pathological lesions are induced when mature morphogen (e.g., OP-1, 20 µg) is administered daily to normal growing rats for 21 consecutive days. Moreover, 10 µg systemic injections of morphogen (e.g., OP-1) injected daily for 10 days into normal newborn mice does not produce any gross abnormalities.

Where morphogens are administered systemically, in the methods of the present invention, preferably a large volume loading dose is used at the start of the treatment. The treatment then is continued with a maintenance dose. Further administration then can be determined by monitoring at intervals the levels of the morphogen in the blood.

## Claims

1. An isolated multimeric protein complex comprising:
a pair of protein subunits associated to form a protein dimer having morphogenic activity.
each of said subunits comprising at least a 100 amino acid sequence having at least 70% amino acid sequence homology with the C-terminal seven cysteine domain of human OP-1, residues 330-431 of SEQ.ID No. 1,
at least one of said subunits comprising a mature form of a subunit of a member of the morphogen family, or an amino acid variant thereof, noncovalently complexed with,
a peptide consisting of a pro region selected from sequence defined by residues 30-48, 30-292 and 48-292 of sequence ID No.1 to form a complex which is more soluble in aqueous solvents than the uncomplexed pair of subunits, said complex eliciting morphogenic activity in vivo.

2. A complex as claimed in claim 1, wherein each of said subunits comprises at least a 100 amino acid sequence having an amino acid sequence defined either by Generic Sequence 7, SEQ.ID No. 20, or by Generic Sequence 8, SEQ.ID No. 21, or by OPX, SEQ.ID NO. 22.

3. A complex as claimed in either claim 1 or claim 2, wherein both said subunits comprise a mature form of a subunit of a member of the morphogen family, or an amino acid variant thereof, each said subunit being noncovalently complexed with said peptide.

4. A complex as claimed in claim 3, wherein each said subunit is the mature form of human OP -1, or an amino acid variant thereof, and optionally said peptide comprises part or all of the pro region of human OP1, BMP2, or BMP4, or an amino acid sequence variant thereof.

5. A complex as claimed in claim 1, wherein said subunit comprises the mature form of a subunit defined by any of the sequences of Seq.ID No. 5-19.

6. A complex as claimed in claim 1, wherein one subunit of said dimer comprises residues 330-431 of Seq.ID No. 1 (OP1) and the other said subunit comprises residues 295-396 of Seq.ID No. 10 (BMP2) or residues 309-408 of Seq.ID No. 11 (BMP4), including amino acid variants thereof.

7. A complex as claimed in any preceding claim, wherein said subunit comprises an amino acid sequence of at least two different morphogen family members.

8. A complex as claimed in claim 1, further comprising a molecule that enhances the stability of said complex.

9. A therapeutic composition comprising the multimeric protein complex as claimed in any one of claims 1-8 and optionally including a cofactor, such as a symptom relieving cofactor.

10. A kit for diagnosing a tissue disorder or evaluating the efficacy of a therapy to regenerate lost or damaged tissue in a mammal, the kit comprising:
(a) means for capturing a cell or fluid, e.g. serum sample from said mammal,
(b) an antibody that interacts specifically with a soluble morphogenic complex in said sample, said soluble morphogenic complex being according to any one of claims 1-8, wherein said antibody discriminates between said soluble morphogenic complex and the mature morphogen dimer.

11. A kit as claimed in claim 10, wherein the quantity of said morphogen is detected by either (a) an immunoassay, or (b) an antibody that distinguishes a soluble morphogen complex from a mature morphogen in a sample fluid.

12. A kit as claimed in either claim 10 or 11, wherein said tissue disorder is a bone tissue disorder, for example osteosarcoma, osteoporosis or Paget's disease.

13. A method of evaluating the status of a tissue, or of evaluating the efficacy of a therapy to regenerate lost or damaged tissue in a mammal, or of diagnosing a tissue disorder in a mammal, the method comprising the step of comparing the quantity of morphogen in a body fluid sample with the quantity of morphogen in a control sample, wherein said quantity of morphogen is detected by using an antibody that distinguishes the multimeric complex of claims 1 to 8 from a mature morphogen in a fluid sample.

14. A method of evaluating the status of a tissue, or of evaluating the efficacy of a therapy to regenerate lost or damaged tissue in a mammal, or of diagnosing a tissue disorder in a mammal, the method comprising the step of detecting the presence of an antibody that binds the multimeric protein complex of claims 1 to 8 in a tissue or body fluid sample, wherein the binding antibody discriminates between said complex and the mature morphogen dimer.

## Patentansprüche

1. Isolierter multimerer Proteinkomplex umfassend:
ein zur Bildung eines Protein-Dimers mit morphogener Aktivität assoziiertes Paar von Protein-Untereinheiten,
wobei jede der genannten Untereinheiten mindestens eine Sequenz aus 100 Aminosäuren umfasst, die mindestens eine 70%ige Aminosäuresequenz-Homologie mit der C-terminalen Domäne mit sieben Cysteinen des humanen OP-1, Resten 330-431 von SEQ ID NO 1 aufweist,
wobei mindestens eine von genannten Untereinheiten eine mature Form einer Untereinheit eines Mitglieds aus der Morphogen-Familie oder eine Aminosäure-Variante davon umfasst,
nicht kovalent komplexiert mit
einem Peptid, das aus einer Proregion besteht, ausgewählt aus Sequenzen, die durch Reste 30-48, 30-292 und 48-292 von SEQ ID NO 1 definiert sind, um einen Komplex zu bilden, der in wässrigen Lösungsmitteln löslicher ist als der nicht komplexierte Teil der Untereinheiten, wobei der genannte Komplex morphogene Aktivität *in vivo* hervorruft.

2. Komplex nach Anspruch 1, worin jede der genannten Untereinheiten mindestens eine Sequenz aus 100 Aminosäuren umfasst, die eine Aminosäuresequenz aufweist, die entweder durch die generische Sequenz 7, SEQ ID NO 20 oder durch die generische Sequenz 8, SEQ ID NO 21 oder durch OPX, SEQ ID NO 22, definiert ist.

3. Komplex nach Anspruch 1 oder 2, worin beide genannten Untereinheiten eine mature Form einer Untereinheit eines Mitglieds der Morphogen-Familie oder eine Aminosäure-Variante davon umfassen, wobei jede genannte Untereinheit nicht kovalent mit genanntem Peptid komplexiert ist.

4. Komplex nach Anspruch 3, worin jede genannte Untereinheit die mature Form von humanem OP-1 oder eine Aminosäure-Variante davon darstellt und genanntes Peptid gegebenenfalls einen Teil der oder die gesamte Proregion von humanem OP1, BMP2 oder BMP4 oder eine Variante einer Aminosäuresequenz davon umfasst.

5. Komplex nach Anspruch 1, worin die genannte Untereinheit die mature Form einer Untereinheit umfasst, die durch jedwede der Sequenzen von SEQ ID NO 5-19 definiert ist.

6. Komplex nach Anspruch 1, worin eine Untereinheit von genanntem Dimer Reste 330-431 von SEQ ID NO 1 (OP1) umfasst und die andere genannte Untereinheit Reste 295-396 von SEQ ID NO 10 (BMP2) oder Reste 309-408 von SEQ ID NO 11 (BMP4), einschließlich Aminosäure-Varianten davon umfasst.

7. Komplex nach einem der vorangehenden Ansprüche, worin die genannte Untereinheit eine Aminosäuresequenz von mindestens zwei verschiedenen Mitgliedern der Morphogen-Familie umfasst.

8. Komplex nach Anspruch 1 weiter umfassend ein Molekül, das die Stabilität von genanntem Komplex verstärkt.

9. Therapeutische Zusammensetzung, die den multimeren Proteinkomplex nach einem der Ansprüche 1-8 umfasst und gegebenenfalls einen Cofaktor, wie zum Beispiel einen symptomlindernden Cofaktor, einschließt.

10. Kit zum Diagnostizieren einer Gewebeerkrankung oder zur Bewertung der Wirksamkeit einer Therapie zur Regeneration von verlorenem oder geschädigtem Gewebe in einem Säuger, wobei das Kit Folgendes umfasst:
(a) Mittel zum Einfangen einer Zelle oder Flüssigkeit, z. B. einer Serumprobe von genanntem Säuger,
(b) einen Antikörper, der spezifisch mit einem löslichen morphogenen Komplex in genannter Probe interagiert, wobei der genannte morphogene Komplex nach einem der Ansprüche 1 - 8 ist, worin genannter Antikörper zwischen genanntem löslichem, morphogenem Komplex und dem maturen Morphogen-Dimer diskriminiert.

11. Kit nach Anspruch 10, worin die Menge von genanntem Morphogen durch entweder (a) einen Immunoassay oder (b) einen Antikörper, der einen löslichen Morphogen-Komplex von einem maturen Morphogen in einer Probenflüssigkeit unterscheidet.

12. Kit nach einem der Ansprüche 10 oder 11, worin genannte Gewebeerkrankung eine Knochengewebserkrankung, zum Beispiel ein Osteosarkom, Osteoporose oder die Paget-Krankheit darstellt.

13. Verfahren zur Bewertung des Status eines Gewebes oder zur Bewertung der Wirksamkeit einer Therapie zur Regeneration von verlorenem oder geschädigtem Gewebe in einem Säuger oder zum Diagnostizieren einer Gewebeerkrankung in einem Säuger, wobei das Verfahren den Schritt zum Vergleich der Morphogen-Menge in einer Körperflüssigkeitsprobe mit der Morphogen-Menge in einer Kontrollprobe umfasst, worin genannte Morphogen-Menge unter Verwendung eines Antikörpers, der den multimeren Komplex nach Ansprüchen 1 bis 8 von einem maturen Morphogen in einer Flüssigkeitsprobe unterscheidet, nachgewiesen wird.

14. Verfahren zur Bewertung des Status eines Gewebes oder zur Bewertung der Wirksamkeit einer Therapie zur Regeneration von verlorenem oder geschädigtem Gewebe in einem Säuger oder zum Diagnostizieren einer Gewebeerkrankung in einem Säuger, wobei das Verfahren den Schritt zum Nachweis des Vorliegens eines Antikörpers umfasst, der den multimeren Proteinkomplex nach Ansprüchen 1 bis 8 in einer Gewebe- oder Körperflüssigkeitsprobe bindet, worin der bindende Antikörper zwischen genanntem Komplex und dem maturen Morphogen-Dimer diskriminiert.

## Revendications

1. Complexe protéique multimère isolé comprenant :
une paire de sous-unités protéiques associées pour former un dimère protéique ayant une activité morphogène.
chacune desdites sous-unités comprenant au moins une séquence de 100 acides aminés ayant une homologie de ces acides aminés d'au moins 70 % avec le domaine C-terminal à sept cystéines de l'OP-1 humaine, résidus 330-431 de la SEQ. ID n° 1.
au moins une desdites sous-unités comprenant une forme mature d'une sous-unité d'un membre de la famille des protéines morphogènes, ou une variante au niveau acides aminés de celle-ci, complexée de manière non covalente avec,
un peptide consistant en une région « pro » sélectionnée à partir de séquences définies par les résidus 30-48, 30-292 et 48-292 de la séquence ID n° 1 pour former un complexe qui est plus soluble dans les solvants aqueux que la partie non complexée des sous-unités, ledit complexe induisant in vivo une activité morphogène.

2. Complexe selon la revendication 1, dans lequel chacune desdites sous-unités comprend au moins une séquence de 100 acides aminés ayant une séquence d'acides aminés définie soit par la Generic Sequence 7, SEQ. ID n° 20, soit par la Generic Sequence 8, SEQ. ID n° 21, soit par OPX, SEQ. ID n° 22.

3. Complexe selon la revendication 1 ou la revendication 2, dans lequel lesdites sous-unités comprennent toutes deux une forme mature d'une sous-unité d'un membre de la famille des protéines morphogènes, ou une variante au niveau acides aminés de celle-ci, chaque dite sous-unité étant complexée de manière non covalente avec ledit peptide.

4. Complexe selon la revendication 3, dans lequel chacune desdites sous-unités est la forme mature de l'OP-1 humaine, ou une variante au niveau acides aminés de celle-ci, et optionnellement ledit peptide comprend une partie ou la totalité de la région « pro » de l'OP1, de la BMP2 ou de la BMP4 humaines, ou une variante au niveau séquence d'acides aminés de celle-ci.

5. Complexe selon la revendication 1, dans lequel ladite sous-unité comprend la forme mature d'une sous-unité définie par n'importe laquelle des séquences de la Seq. ID n° 5-19.

6. Complexe selon la revendication 1, dans lequel une sous-unité dudit dimère comprend les résidus 330-431 de la Seq. ID n° 1 (0P1) et l'autre dite sous-unité comprend les résidus 295-396 de la Seq. ID n° 10 (BMP2) ou les résidus 309-408 de la Seq. ID n° 11 (BMP4), y compris les variantes au niveau acides aminés de celles-ci.

7. Complexe selon n'importe quelle revendication précédente, dans lequel ladite sous-unité comprend une séquence d'acides aminés d'au moins deux membres différents de la famille des protéines morphogènes.

8. Complexe selon la revendication 1, comprenant également une molécule qui améliore la stabilité dudit complexe.

9. Composé thérapeutique comprenant le complexe de protéines multimères comme revendiqué dans n'importe laquelle des revendications 1 à 8 et comprenant optionnellement un cofacteur tel qu'un cofacteur de soulagement des symptômes.

10. Kit pour diagnostiquer un trouble tissulaire ou pour évaluer l'efficacité d'un traitement pour régénérer des tissus perdus ou endommagés chez un mammifère, le kit comprenant :
(a) un moyen pour capturer une cellule ou un fluide, p.ex. un échantillon de sérum dudit mammifère,
(b) un anticorps qui interagit spécifiquement avec un complexe morphogène soluble dans ledit échantillon, ledit complexe morphogène soluble étant selon n'importe laquelle des revendications 1 à 8, ledit anticorps étant capable de distinguer ledit complexe morphogène soluble du dimère protéines morphogènes mature.

11. Kit selon la revendication 10, la quantité de ladite protéine morphogène étant détectée (a) soit par un immuno-essai, soit (b) par un anticorps capable de distinguer un complexe morphogène soluble d'une protéine morphogène mature dans un échantillon de fluide.

12. Kit selon n'importe laquelle des revendications 10 ou 11, ledit trouble tissulaire étant un trouble du tissu osseux, par exemple ostéosarcome, ostéoporose ou maladie de Paget.

13. Méthode d'évaluation de l'état d'un tissu, ou d'évaluation de l'efficacité d'un traitement pour régénérer des tissus perdus ou endommagés chez un mammifère, ou de diagnostic d'un trouble tissulaire chez un mammifère, la méthode comprenant l'étape consistant à comparer la quantité de protéines morphogènes dans un échantillon de fluide corporel avec la quantité de protéines morphogènes dans un échantillon de contrôle, ladite quantité de protéines morphogènes étant détectée en utilisant un anticorps qui distingue le complexe multimère des revendications 1 à 8 d'une protéine morphogène mature dans un échantillon de fluide.

14. Méthode d'évaluation de l'état d'un tissu, ou d'évaluation de l'efficacité d'un traitement pour régénérer des tissus perdus ou endommagés chez un mammifère, ou de diagnostic d'un trouble tissulaire chez un mammifère, la méthode comprenant l'étape consistant à détecter la présence d'un anticorps qui se lie au complexe protéique multimère des revendications 1 à 8 dans un échantillon de tissu ou de fluide corporel, l'anticops liant distinguant ledit complexe du dimère protéines morphogènes mature.


### SEQUENCE LISTING

(1) GENERAL INFORMATION:
   (i) APPLICANT:
      (A) NAME: CREATIVE BIOMOLECULES, INC.
      (B) STREET: 35 SOUTH STREET
      (C) CITY: HOPKINTON
      (D) STATE: MA
      (E) COUNTRY: USA
      (F) POSTAL CODE (ZIP): 01748
      (G) TELEPHONE: 1-508-435-9001
      (H) TELEFAX: 1-508-435-0454
      (I) TELEX:
   (ii) TITLE OF INVENTION: NOVEL MORPHOGENIC PROTEIN COMPOSITIONS OF MATTER
   (iii) NUMBER OF SEQUENCES: 23
   (iv) CORRESPONDENCE ADDRESS:
      (A) ADDRESSEE: PATENT ADMINISTRATOR/CREATIVE BIOMOLECULES, INC.
      (B) STREET: 35 SOUTH STREET
      (C) CITY: HOPKINTON
      (D) STATE: MA
      (E) COUNTRY: USA
      (F) ZIP: 01748
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: KELLEY, ROBIN, D.
      (B) REGISTRATION NUMBER: 34,637
      (C) REFERENCE/DOCKET NUMBER: CRP-081CP
(2) INFORMATION FOR SEQ ID NO:1:
   (i) SEQUENCE CHARACTERISTICS:
      (A) LENGTH: 1822 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: CDS
      (B) LOCATION: 49..1341
      (C) IDENTIFICATION METHOD: experimental
      (D) OTHER INFORMATION: /function= "OSTEOGENIC PROTEIN"
         /product= "OP1"
         /evidence= EXPERIMENTAL
         /standard_name= "OP1"
   (xi) SEQUENCE DESCRIPTION: SEQ ID NO:1:
   GGTGCGGGCC CGGAGCCCGG AGCCCGGGTA GCGCGTAGAG CCGGCGCG ATG CAC GTG         57
                                                        Met His Val
                                                          1
   CGC TCA CTG CGA GCT GCG GCG CCG CAC AGC TTC GTG GCG CTC TGG GCA         105
   Arg Ser Leu Arg Ala Ala Ala Pro His Ser Phe Val Ala Leu Trp Ala
         5                  10                  15
   CCC CTG TTC CTG CTG CGC TCC GCC CTG GCC GAC TTC AGC CTG GAC AAC         153
   Pro Leu Phe Leu Leu Arg Ser Ala Leu Ala Asp Phe Ser Leu Asp Asn
    20                  25                  30                  35
   GAG GTG CAC TCG AGC TTC ATC CAC CGG CGC CTC CGC AGC CAG GAG CGG         201
   Glu Val His Ser Ser Phe Ile His Arg Arg Leu Arg Ser Gln Glu Arg
                    40                  45                  50
   CGG GAG ATG CAG CGC GAG ATC CTC TCC ATT TTG GGC TTG CCC CAC CGC         249
   Arg Glu Met Gln Arg Glu Ile Leu Ser Ile Leu Gly Leu Pro His Arg
                55                  60                  65
   CCG CGC CCG CAC CTC CAG GGC AAG CAC AAC TCG GCA CCC ATG TTC ATG      297
   Pro Arg Pro His Leu Gln Gly Lys His Asn Ser Ala Pro Met Phe Met
            70                  75                  80
   CTG GAC CTG TAC AAC GCC ATG GCG GTG GAG GAG GGC GGC GGG CCC GGC      345
   Leu Asp Leu Tyr Asn Ala Met Ala Val Glu Glu Gly Gly Gly Pro Gly
        85                  90                  95
   GGC CAG GGC TTC TCC TAC CCC TAC AAG GCC GTC TTC AGT ACC CAG GGC      393
   Gly Gln Gly Phe Ser Tyr Pro Tyr Lys Ala Val Phe Ser Thr Gln Gly
   100                 105                 110                 115
   CCC CCT CTG GCC AGC CTG CAA GAT AGC CAT TTC CTC ACC GAC GCC GAC      441
   Pro Pro Leu Ala Ser Leu Gln Asp Ser His Phe Leu Thr Asp Ala Asp
                   120                 125                 130
   ATG GTC ATG AGC TTC GTC AAC CTC GTG GAA CAT GAC AAG GAA TTC TTC      489
   Met Val Met Ser Phe Val Asn Leu Val Glu His Asp Lys Glu Phe Phe
               135                 140                 145
   CAC CCA CGC TAC CAC CAT CGA GAG TTC CGG TTT GAT CTT TCC AAG ATC      537
   His Pro Arg Tyr His His Arg Glu Phe Arg Phe Asp Leu Ser Lys Ile
           150                 155                 160
   CCA GAA GGG GAA GCT GTC ACG GCA GCC GAA TTC CGG ATC TAC AAG GAC      585
   Pro Glu Gly Glu Ala Val Thr Ala Ala Glu Phe Arg Ile Tyr Lys Asp
       165                 170                 175
   TAC ATC CGG GAA CGC TTC GAC AAT GAG ACG TTC CGG ATC AGC GTT TAT      633
   Tyr Ile Arg Glu Arg Phe Asp Asn Glu Thr Phe Arg Ile Ser Val Tyr
   180                 185                 190                 195
   CAG GTG CTC CAG GAG CAC TTG GGC AGG GAA TCG GAT CTC TTC CTG CTC      681
   Gln Val Leu Gln Glu His Leu Gly Arg Glu Ser Asp Leu Phe Leu Leu
                   200                 205                 210
   GAC AGC CGT ACC CTC TGG GCC TCG GAG GAG GGC TGG CTG GTG TTT GAC      729
   Asp Ser Arg Thr Leu Trp Ala Ser Glu Glu Gly Trp Leu Val Phe Asp
               215                 220                 225
   ATC ACA GCC ACC AGC AAC CAC TGG GTG GTC AAT CCG CGG CAC AAC CTG      777
   Ile Thr Ala Thr Ser Asn His Trp Val Val Asn Pro Arg His Asn Leu
           230                 235                 240
   GGC CTG CAG CTC TCG GTG GAG ACG CTG GAT GGG CAG AGC ATC AAC CCC      825
   Gly Leu Gln Leu Ser Val Glu Thr Leu Asp Gly Gln Ser Ile Asn Pro
       245                 250                 255
   AAG TTG GCG GGC CTG ATT GGG CGG CAC GGG CCC CAG AAC AAG CAG CCC      873
   Lys Leu Ala Gly Leu Ile Gly Arg His Gly Pro Gln Asn Lys Gln Pro
   260                 265                 270                 275
   TTC ATG GTG GCT TTC TTC AAG GCC ACG GAG GTC CAC TTC CGC AGC ATC      921
   Phe Met Val Ala Phe Phe Lys Ala Thr Glu Val His Phe Arg Ser Ile
                   280                 285                 290
   CGG TCC ACG GGG AGC AAA CAG CGC AGC CAG AAC CGC TCC AAG ACG CCC      969
   Arg Ser Thr Gly Ser Lys Gln Arg Ser Gln Asn Arg Ser Lys Thr Pro
               295                 300                 305
   AAG AAC CAG GAA GCC CTG CGG ATG GCC AAC GTG GCA GAG AAC AGC AGC     1017
   Lys Asn Gln Glu Ala Leu Arg Met Ala Asn Val Ala Glu Asn Ser Ser
           310                 315                 320
   AGC GAC CAG AGG CAG GCC TGT AAG AAG CAC GAG CTG TAT GTC AGC TTC     1065
   Ser Asp Gln Arg Gln Ala Cys Lys Lys His Glu Leu Tyr Val Ser Phe
       325                 330                 335
   CGA GAC CTG GGC TGG CAG GAC TGG ATC ATC GCG CCT GAA GGC TAC GCC     1113
   Arg Asp Leu Gly Trp Gln Asp Trp Ile Ile Ala Pro Glu Gly Tyr Ala
   340                 345                 350                 355
   GCC TAC TAC TGT GAG GGG GAG TGT GCC TTC CCT CTG AAC TCC TAC ATG     1161
   Ala Tyr Tyr Cys Glu Gly Glu Cys Ala Phe Pro Leu Asn Ser Tyr Met
                   360                 365                 370
   AAC GCC ACC AAC CAC GCC ATC GTG CAG ACG CTG GTC CAC TTC ATC AAC     1209
   Asn Ala Thr Asn His Ala Ile Val Gln Thr Leu Val His Phe Ile Asn
               375                 380                 385
   CCG GAA ACG GTG CCC AAG CCC TGC TGT GCG CCC ACG CAG CTC AAT GCC     1257
   Pro Glu Thr Val Pro Lys Pro Cys Cys Ala Pro Thr Gln Leu Asn Ala
           390                 395                 400
   ATC TCC GTC CTC TAC TTC GAT GAC AGC TCC AAC GTC ATC CTG AAG AAA     1305
   Ile Ser Val Leu Tyr Phe Asp Asp Ser Ser Asn Val Ile Leu Lys Lys
       405                 410                 415
   TAC AGA AAC ATG GTG GTC CGG GCC TGT GGC TGC CAC TAGCTCCTCC          1351
   Tyr Arg Asn Met Val Val Arg Ala Cys Gly Cys His
   420                 425                 430
   GAGAATTCAG ACCCTTTGGG GCCAAGTTTT TCTGGATCCT CCATTGCTCG CCTTGGCCAG   1411
   GAACCAGCAG ACCAACTGCC TTTTGTGAGA CCTTCCCCTC CCTATCCCCA ACTTTAAAGG   1471
   TGTGAGAGTA TTAGGAAACA TGAGCAGCAT ATGGCTTTTG ATCAGTTTTT CAGTGGCAGC   1531
   ATCCAATGAA CAAGATCCTA CAAGCTGTGC AGGCAAAACC TAGCAGGAAA AAAAAACAAC    1591
   GCATAAAGAA AAATGGCCGG GCCAGGTCAT TGGCTGGGAA GTCTCAGCCA TGCACGGACT    1651
   CGTTTCCAGA GGTAATTATG AGCGCCTACC AGCCAGGCCA CCCAGCCGTG GGAGGAAGGG    1711
   GGCGTGGCAA GGGGTGGGCA CATTGGTGTC TGTGCGAAAG GAAAATTGAC CCGGAAGTTC    1771
   CTGTAATAAA TGTCACAATA AAACGAATGA ATGAAAAAAA AAAAAAAAAA A             1822
(2) INFORMATION FOR SEQ ID NO:2:
   (i) SEQUENCE CHARACTERISTICS:
      (A) LENGTH: 431 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Arg Ser Gln Asn Arg Ser
   290 295 300
   Lys Thr Pro Lys Asn Gln Glu Ala Leu Arg Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Arg Asn Met Val Val Arg Ala Cys Gly Cys His
   420 425 430
(2) INFORMATION FOR SEQ ID NO:3
   (i) SEQUENCE CHARACTERISTICS:
      (A) LENGTH: 1873 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 104..1393
      (D) OTHER INFORMATION: /function= "OSTEOGENIC PROTEIN"
         /product= "HOP1"
         /note= "MOP1 CDNA"
   (xi) SEQUENCE DESCRIPTION: SEQ ID NO:3:
   CTGCAGCAAG TGACCTCGGG TCGTGGACCG CTGCCCTGCC CCCTCCGCTG CCACCTGGGG       60
   CGGCGCGGGC CCGGTGCCCC GGATCGCGCG TAGAGCCGGC GCG ATG CAC GTG CGC       115
                                                   Met His Val Arg
                                                     1
   TCG CTG CGC GCT GCG GCG CCA CAC AGC TTC GTG GCG CTC TGG GCG CCT      163
   Ser Leu Arg Ala Ala Ala Pro His Ser Phe Val Ala Leu Trp Ala Pro
     5                  10                  15                  20
   CTG TTC TTG CTG CGC TCC GCC CTG GCC GAT TTC AGC CTG GAC AAC GAG      211
   Leu Phe Leu Leu Arg Ser Ala Leu Ala Asp Phe Ser Leu Asp Asn Glu
                    25                  30                  35
   GTG CAC TCC AGC TTC ATC CAC CGG CGC CTC CGC AGC CAG GAG CGG CGG      259
   Val His Ser Ser Phe Ile His Arg Arg Leu Arg Ser Gln Glu Arg Arg
                40                  45                  50
   GAG ATG CAG CGG GAG ATC CTG TCC ATC TTA GGG TTG CCC CAT CGC CCG      307
   Glu Met Gln Arg Glu Ile Leu Ser Ile Leu Gly Leu Pro His Arg Pro
            55                  60                  65
   CGC CCG CAC CTC CAG GGA AAG CAT AAT TCG GCG CCC ATG TTC ATG TTG      355
   Arg Pro His Leu Gln Gly Lys His Asn Ser Ala Pro Met Phe Met Leu
        70                  75                  80
   GAC CTG TAC AAC GCC ATG GCG GTG GAG GAG AGC GGG CCG GAC GGA CAG      403
   Asp Leu Tyr Asn Ala Met Ala Val Glu Glu Ser Gly Pro Asp Gly Gln
    85                  90                  95                 100
   GGC TTC TCC TAC CCC TAC AAG GCC GTC TTC AGT ACC CAG GGC CCC CCT      451
   Gly Phe Ser Tyr Pro Tyr Lys Ala Val Phe Ser Thr Gln Gly Pro Pro
                   105                 110                 115
   TTA GCC AGC CTG CAG GAC AGC CAT TTC CTC ACT GAC GCC GAC ATG GTC      499
   Leu Ala Ser Leu Gln Asp Ser His Phe Leu Thr Asp Ala Asp Met Val
               120                 125                 130
   ATG AGC TTC GTC AAC CTA GTG GAA CAT GAC AAA GAA TTC TTC CAC CCT      547
   Met Ser Phe Val Asn Leu Val Glu His Asp Lys Glu Phe Phe His Pro
           135                 140                 145
   CGA TAC CAC CAT CGG GAG TTC CGG TTT GAT CTT TCC AAG ATC CCC GAG      595
   Arg Tyr His His Arg Glu Phe Arg Phe Asp Leu Ser Lys Ile Pro Glu
       150                 155                 160
   GGC GAA CGG GTG ACC GCA GCC GAA TTC AGG ATC TAT AAG GAC TAC ATC      643
   Gly Glu Arg Val Thr Ala Ala Glu Phe Arg Ile Tyr Lys Asp Tyr Ile
   165                 170                 175                 180
   CGG GAG CGA TTT GAC AAC GAG ACC TTC CAG ATC ACA GTC TAT CAG GTG      691
   Arg Glu Arg Phe Asp Asn Glu Thr Phe Gln Ile Thr Val Tyr Gln Val
                   185                 190                 195
   CTC CAG GAG CAC TCA GGC AGG GAG TCG GAC CTC TTC TTG CTG GAC AGC      739
   Leu Gln Glu His Ser Gly Arg Glu Ser Asp Leu Phe Leu Leu Asp Ser
               200                 205                 210
   CGC ACC ATC TGG GCT TCT GAG GAG GGC TGG TTG GTG TTT GAT ATC ACA      787
   Arg Thr Ile Trp Ala Ser Glu Glu Gly Trp Leu Val Phe Asp Ile Thr
           215                 220                 225
   GCC ACC AGC AAC CAC TGG GTG GTC AAC CCT CGG CAC AAC CTG GGC TTA      835
   Ala Thr Ser Asn His Trp Val Val Asn Pro Arg His Asn Leu Gly Leu
       230                 235                 240
   CAG CTC TCT GTG GAG ACC CTG GAT GGG CAG AGC ATC AAC CCC AAG TTG      883
   Gln Leu Ser Val Glu Thr Leu Asp Gly Gln Ser Ile Asn Pro Lys Leu
   245                 250                 255                 260
   GCA GGC CTG ATT GGA CGG CAT GGA CCC CAG AAC AAG CAA CCC TTC ATG       931
   Ala Gly Leu Ile Gly Arg His Gly Pro Gln Asn Lys Gln Pro Phe Met
                   265                 270                 275
   GTG GCC TTC TTC AAG GCC ACG GAA GTC CAT CTC CGT AGT ATC CGG TCC       979
   Val Ala Phe Phe Lys Ala Thr Glu Val His Leu Arg Ser Ile Arg Ser
               280                 285                 290
   ACG GGG GGC AAG CAG CGC AGC CAG AAT CGC TCC AAG ACG CCA AAG AAC      1027
   Thr Gly Gly Lys Gln Arg Ser Gln Asn Arg Ser Lys Thr Pro Lys Asn
           295                 300                 305
   CAA GAG GCC CTG AGG ATG GCC AGT GTG GCA GAA AAC AGC AGC AGT GAC      1075
   Gln Glu Ala Leu Arg Met Ala Ser Val Ala Glu Asn Ser Ser Ser Asp
       310                 315                 320
   CAG AGG CAG GCC TGC AAG AAA CAT GAG CTG TAC GTC AGC TTC CGA GAC      1123
   Gln Arg Gln Ala Cys Lys Lys His Glu Leu Tyr Val Ser Phe Arg Asp
   325                 330                 335                 340
   CTT GGC TGG CAG GAC TGG ATC ATT GCA CCT GAA GGC TAT GCT GCC TAC      1171
   Leu Gly Trp Gln Asp Trp Ile Ile Ala Pro Glu Gly Tyr Ala Ala Tyr
                   345                 350                 355
   TAC TGT GAG GGA GAG TGC GCC TTC CCT CTG AAC TCC TAC ATG AAC GCC      1219
   Tyr Cys Glu Gly Glu Cys Ala Phe Pro Leu Asn Ser Tyr Met Asn Ala
               360                 365                 370
   ACC AAC CAC GCC ATC GTC CAG ACA CTG GTT CAC TTC ATC AAC CCA GAC      1267
   Thr Asn His Ala Ile Val Gln Thr Leu Val His Phe Ile Asn Pro Asp
           375                 380                 385
   ACA GTA CCC AAG CCC TGC TGT GCG CCC ACC CAG CTC AAC GCC ATC TCT      1315
   Thr Val Pro Lys Pro Cys Cys Ala Pro Thr Gln Leu Asn Ala Ile Ser
       390                 395                 400
   GTC CTC TAC TTC GAC GAC AGC TCT AAT GTC ATC CTG AAG AAG TAC AGA      1363
   Val Leu Tyr Phe Asp Asp Ser Ser Asn Val Ile Leu Lys Lys Tyr Arg
   405                 410                 415                 420
   AAC ATG GTG GTC CGG GCC TGT GGC TGC CAC TAGCTCTTCC TGAGACCCTG        1413
   Asn Met Val Val Arg Ala Cys Gly Cys His
                   425                 430
   ACCTTTGCGG GGCCACACCT TTCCAAATCT TCGATGTCTC ACCATCTAAG TCTCTCACTG    1473
   CCCACCTTGG CGAGGAGAAC AGACCAACCT CTCCTGAGCC TTCCCTCACC TCCCAACCGG    1533
   AAGCATGTAA GGGTTCCAGA AACCTGAGCG TGCAGCAGCT GATGAGCGCC CTTTCCTTCT    1593
   GGCACGTGAC GGACAAGATC CTACCAGCTA CCACAGCAAA CGCCTAAGAG CAGGAAAAAT    1653
   GTCTGCCAGG AAAGTGTCCA GTGTCCACAT GGCCCCTGGC GCTCTGAGTC TTTGAGGAGT    1713
   AATCGCAAGC CTCGTTCAGC TGCAGCAGAA GGAAGGGCTT AGCCAGGGTG GGCGCTGGCG    1773
   TCTGTGTTGA AGGGAAACCA AGCAGAAGCC ACTGTAATGA TATGTCACAA TAAAACCCAT    1833
   GAATGAAAAA AAAAAAAAAA AAAAAAAAAA AAAAGAATTC                          1873
(2) INFORMATION FOR SEQ ID NO:4:
   (i) SEQUENCE CHARACTERISTICS:
      (A) LENGTH: 430 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Ser Gly
                   85                  90                  95
   Pro Asp Gly Gln Gly Phe Ser Tyr Pro Tyr Lys Ala Val Phe Ser Thr
               100                 105                 110
   Gln Gly Pro Pro Leu Ala Ser Leu Gln Asp Ser His Phe Leu Thr Asp
           115                 120                 125
   Ala Asp Met Val Met Ser Phe Val Asn Leu Val Glu His Asp Lys Glu
       130                 135                 140
   Phe Phe His Pro Arg Tyr His His Arg Glu Phe Arg Phe Asp Leu Ser
   145 150 155 160
   Lys Ile Pro Glu Gly Glu Arg Val Thr Ala Ala Glu Phe Arg Ile Tyr
   165 170 175
   Lys Asp Tyr Ile Arg Glu Arg Phe Asp Asn Glu Thr Phe Gln Ile Thr
   180 185 190
   Val Tyr Gln Val Leu Gln Glu His Ser Gly Arg Glu Ser Asp Leu Phe
   195 200 205
   Leu Leu Asp Ser Arg Thr Ile Trp Ala Ser Glu Glu Gly Trp Leu Val
   210 215 220
   Phe Asp Ile Thr Ala Thr Ser Asn His Trp Val Val Asn Pro Arg His
   225 230 235 240
   Asn Leu Gly Leu Gln Leu Ser Val Glu Thr Leu Asp Gly Gln Ser Ile
   245 250 255
   Asn Pro Lys Leu Ala Gly Leu Ile Gly Arg His Gly Pro Gln Asn Lys
   260 265 270
   Gln Pro Phe Met Val Ala Phe Phe Lys Ala Thr Glu Val His Leu Arg
   275 280 285
   Ser Ile Arg Ser Thr Gly Gly Lys Gln Arg Ser Gln Asn Arg Ser Lys
   290 295 300
   Thr Pro Lys Asn Gln Glu Ala Leu Arg Met Ala Ser Val Ala Glu Asn
   305 310 315 320
   Ser Ser Ser Asp Gln Arg Gln Ala Cys Lys Lys His Glu Leu Tyr Val
   325 330 335
   Ser Phe Arg Asp Leu Gly Trp Gln Asp Trp Ile Ile Ala Pro Glu Gly
   340 345 350
   Tyr Ala Ala Tyr Tyr Cys Glu Gly Glu Cys Ala Phe Pro Leu Asn Ser
   355 360 365
   Tyr Met Asn Ala Thr Asn His Ala Ile Val Gln Thr Leu Val His Phe
   370 375 380
   Ile Asn Pro Asp Thr Val Pro Lys Pro Cys Cys Ala Pro Thr Gln Leu
   385 390 395 400
   Asn Ala Ile Ser Val Leu Tyr Phe Asp Asp Ser Ser Asn Val Ile Leu
   405 410 415
   Lys Lys Tyr Arg Asn Met Val Val Arg Ala Cys Gly Cys His
   420 425 430
(2) INFORMATION FOR SEQ ID NO:5:
   (i) SEQUENCE CHARACTERISTICS:
      (A) LENGTH: 1723 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
      (F) TISSUE TYPE: HIPPOCAMPUS
   (ix) FEATURE:
      (A) NAME/KEY: CDS
      (B) LOCATION: 490..1696
      (D) OTHER INFORMATION: /function= "OSTEOGENIC PROTEIN"
         /product= "hOP2-PP"
         /note= "hOP2 (cDNA)"
   (xi) SEQUENCE DESCRIPTION: SEQ ID NO:5:
   GGCGCCGGCA GAGCAGGAGT GGCTGGAGGA GCTGTGGTTG GAGCAGGAGG TGGCACGGCA      60
   GGGCTGGAGG GCTCCCTATG AGTGGCGGAG ACGGCCCAGG AGGCGCTGGA GCAACAGCTC     120
   CCACACCGCA CCAAGCGGTG GCTGCAGGAG CTCGCCCATC GCCCCTGCGC TGCTCGGACC     180
   GCGGCCACAG CCGGACTGGC GGGTACGGCG GCGACAGAGG CATTGGCCGA GAGTCCCAGT     240
   CCGCAGAGTA GCCCCGGCCT CGAGGCGGTG GCGTCCCGGT CCTCTCCGTC CAGGAGCCAG     300
   GACAGGTGTC GCGCGGCGGG GCTCCAGGGA CCGCGCCTGA GGCCGGCTGC CCGCCCGTCC     360
   CGCCCCGCCC CGCCGCCCGC CGCCCGCCGA GCCCAGCCTC CTTGCCGTCG GGGCGTCCCC     420
   AGGCCCTGGG TCGGCCGCGG AGCCGATGCG CGCCCGCTGA GCGCCCCAGC TGAGCGCCCC     480
   CGGCCTGCC ATG ACC GCG CTC CCC GGC CCG CTC TGG CTC CTG GGC CTG         528
             Met Thr Ala Leu Pro Gly Pro Leu Trp Leu Leu Gly Leu
               1               5                  10
   GCG CTA TGC GCG CTG GGC GGG GGC GGC CCC GGC CTG CGA CCC CCG CCC      576
   Ala Leu Cys Ala Leu Gly Gly Gly Gly Pro Gly Leu Arg Pro Pro Pro
        15                  20                  25
   GGC TGT CCC CAG CGA CGT CTG GGC GCG CGC GAG CGC CGG GAC GTG CAG      624
   Gly Cys Pro Gln Arg Arg Leu Gly Ala Arg Glu Arg Arg Asp Val Gln
    30                  35                  40                  45
   CGC GAG ATC CTG GCG GTG CTC GGG CTG CCT GGG CGG CCC CGG CCC CGC      672
   Arg Glu Ile Leu Ala Val Leu Gly Leu Pro Gly Arg Pro Arg Pro Arg
                    50                  55                  60
   GCG CCA CCC GCC GCC TCC CGG CTG CCC GCG TCC GCG CCG CTC TTC ATG      720
   Ala Pro Pro Ala Ala Ser Arg Leu Pro Ala Ser Ala Pro Leu Phe Met
                65                  70                  75
   CTG GAC CTG TAC CAC GCC ATG GCC GGC GAC GAC GAC GAG GAC GGC GCG      768
   Leu Asp Leu Tyr His Ala Met Ala Gly Asp Asp Asp Glu Asp Gly Ala
            80                  85                  90
   CCC GCG GAG CGG CGC CTG GGC CGC GCC GAC CTG GTC ATG AGC TTC GTT      816
   Pro Ala Glu Arg Arg Leu Gly Arg Ala Asp Leu Val Met Ser Phe Val
        95                 100                 105
   AAC ATG GTG GAG CGA GAC CGT GCC CTG GGC CAC CAG GAG CCC CAT TGG      864
   Asn Met Val Glu Arg Asp Arg Ala Leu Gly His Gln Glu Pro His Trp
   110                 115                 120                 125
   AAG GAG TTC CGC TTT GAC CTG ACC CAG ATC CCG GCT GGG GAG GCG GTC      912
   Lys Glu Phe Arg Phe Asp Leu Thr Gln Ile Pro Ala Gly Glu Ala Val
                   130                 135                 140
   ACA GCT GCG GAG TTC CGG ATT TAC AAG GTG CCC AGC ATC CAC CTG CTC      960
   Thr Ala Ala Glu Phe Arg Ile Tyr Lys Val Pro Ser Ile His Leu Leu
               145                 150                 155
   AAC AGG ACC CTC CAC GTC AGC ATG TTC CAG GTG GTC CAG GAG CAG TCC     1008
   Asn Arg Thr Leu His Val Ser Met Phe Gln Val Val Gln Glu Gln Ser
           160                 165                 170
   AAC AGG GAG TCT GAC TTG TTC TTT TTG GAT CTT CAG ACG CTC CGA GCT     1056
   Asn Arg Glu Ser Asp Leu Phe Phe Leu Asp Leu Gln Thr Leu Arg Ala
       175                 180                 185
   GGA GAC GAG GGC TGG CTG GTG CTG GAT GTC ACA GCA GCC AGT GAC TGC     1104
   Gly Asp Glu Gly Trp Leu Val Leu Asp Val Thr Ala Ala Ser Asp Cys
   190                 195                 200                 205
   TGG TTG CTG AAG CGT CAC AAG GAC CTG GGA CTC CGC CTC TAT GTG GAG      1152
   Trp Leu Leu Lys Arg His Lys Asp Leu Gly Leu Arg Leu Tyr Val Glu
                   210                 215                 220
   ACT GAG GAC GGG CAC AGC GTG GAT CCT GGC CTG GCC GGC CTG CTG GGT      1200
   Thr Glu Asp Gly His Ser Val Asp Pro Gly Leu Ala Gly Leu Leu Gly
               225                 230                 235
   CAA CGG GCC CCA CGC TCC CAA CAG CCT TTC GTG GTC ACT TTC TTC AGG      1248
   Gln Arg Ala Pro Arg Ser Gln Gln Pro Phe Val Val Thr Phe Phe Arg
           240                 245                 250
   GCC AGT CCG AGT CCC ATC CGC ACC CCT CGG GCA GTG AGG CCA CTG AGG      1296
   Ala Ser Pro Ser Pro Ile Arg Thr Pro Arg Ala Val Arg Pro Leu Arg
       255                 260                 265
   AGG AGG CAG CCG AAG AAA AGC AAC GAG CTG CCG CAG GCC AAC CGA CTC      1344
   Arg Arg Gln Pro Lys Lys Ser Asn Glu Leu Pro Gln Ala Asn Arg Leu
   270                 275                 280                 285
   CCA GGG ATC TTT GAT GAC GTC CAC GGC TCC CAC GGC CGG CAG GTC TGC      1392
   Pro Gly Ile Phe Asp Asp Val His Gly Ser His Gly Arg Gln Val Cys
                   290                 295                 300
   CGT CGG CAC GAG CTC TAC GTC AGC TTC CAG GAC CTC GGC TGG CTG GAC      1440
   Arg Arg His Glu Leu Tyr Val Ser Phe Gln Asp Leu Gly Trp Leu Asp
               305                 310                 315
   TGG GTC ATC GCT CCC CAA GGC TAC TCG GCC TAT TAC TGT GAG GGG GAG      1488
   Trp Val Ile Ala Pro Gln Gly Tyr Ser Ala Tyr Tyr Cys Glu Gly Glu
           320                 325                 330
   TGC TCC TTC CCA CTG GAC TCC TGC ATG AAT GCC ACC AAC CAC GCC ATC      1536
   Cys Ser Phe Pro Leu Asp Ser Cys Met Asn Ala Thr Asn His Ala Ile
       335                 340                 345
   CTG CAG TCC CTG GTG CAC CTG ATG AAG CCA AAC GCA GTC CCC AAG GCG      1584
   Leu Gln Ser Leu Val His Leu Met Lys Pro Asn Ala Val Pro Lys Ala
   350                 355                 360                 365
   TGC TGT GCA CCC ACC AAG CTG AGC GCC ACC TCT GTG CTC TAC TAT GAC      1632
   Cys Cys Ala Pro Thr Lys Leu Ser Ala Thr Ser Val Leu Tyr Tyr Asp
                   370                 375                 380
   AGC AGC AAC AAC GTC ATC CTG CGC AAA GCC CGC AAC ATG GTG GTC AAG      1680
   Ser Ser Asn Asn Val Ile Leu Arg Lys Ala Arg Asn Met Val Val Lys
               385                 390                 395
   GCC TGC GGC TGC CAC T GAGTCAGCCC GCCCAGCCCT ACTGCAG                  1723
   Ala Cys Gly Cys His
           400
(2) INFORMATION FOR SEQ ID NO:6:
   (i) SEQUENCE CHARACTERISTICS:
      (A) LENGTH: 402 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Thr Ala Leu Pro Gly Pro Leu Trp Leu Leu Gly Leu Ala Leu Cys
    1              5                   10                  15
   Ala Leu Gly Gly Gly Gly Pro Gly Leu Arg Pro Pro Pro Gly Cys Pro
               20                  25                  30
   Gln Arg Arg Leu Gly Ala Arg Glu Arg Arg Asp Val Gln Arg Glu Ile
           35                  40                  45
   Leu Ala Val Leu Gly Leu Pro Gly Arg Pro Arg Pro Arg Ala Pro Pro
       50                  55                  60
   Ala Ala Ser Arg Leu Pro Ala Ser Ala Pro Leu Phe Met Leu Asp Leu
   65                  70                  75                  80
   Tyr His Ala Met Ala Gly Asp Asp Asp Glu Asp Gly Ala Pro Ala Glu
                   85                  90                  95
   Arg Arg Leu Gly Arg Ala Asp Leu Val Met Ser Phe Val Asn Met Val
               100                 105                 110
   Glu Arg Asp Arg Ala Leu Gly His Gln Glu Pro His Trp Lys Glu Phe
           115                 120                 125
   Arg Phe Asp Leu Thr Gln Ile Pro Ala Gly Glu Ala Val Thr Ala Ala
       130                 135                 140
   Glu Phe Arg Ile Tyr Lys Val Pro Ser Ile His Leu Leu Asn Arg Thr
   145                 150                 155                 160
   Leu His Val Ser Met Phe Gln Val Val Gln Glu Gln Ser Asn Arg Glu
                   165                 170                 175
   Ser Asp Leu Phe Phe Leu Asp Leu Gln Thr Leu Arg Ala Gly Asp Glu
               180                 185                 190
   Gly Trp Leu Val Leu Asp Val Thr Ala Ala Ser Asp Cys Trp Leu Leu
           195                 200                 205
   Lys Arg His Lys Asp Leu Gly Leu Arg Leu Tyr Val Glu Thr Glu Asp
       210                 215                 220
   Gly His Ser Val Asp Pro Gly Leu Ala Gly Leu Leu Gly Gln Arg Ala
   225                 230                 235                 240
   Pro Arg Ser Gln Gln Pro Phe Val Val Thr Phe Phe Arg Ala Ser Pro
                   245                 250                 255
   Ser Pro Ile Arg Thr Pro Arg Ala Val Arg Pro Leu Arg Arg Arg Gln
               260                 265                 270
   Pro Lys Lys Ser Asn Glu Leu Pro Gln Ala Asn Arg Leu Pro Gly Ile
           275                 280                 285
   Phe Asp Asp Val His Gly Ser His Gly Arg Gln Val Cys Arg Arg His
       290                 295                 300
   Glu Leu Tyr Val Ser Phe Gln Asp Leu Gly Trp Leu Asp Trp Val Ile
   305                 310                 315                 320
   Ala Pro Gln Gly Tyr Ser Ala Tyr Tyr Cys Glu Gly Glu Cys Ser Phe
                   325                 330                 335
   Pro Leu Asp Ser Cys Met Asn Ala Thr Asn His Ala Ile Leu Gln Ser
               340                 345                 350
   Leu Val His Leu Met Lys Pro Asn Ala Val Pro Lys Ala Cys Cys Ala
           355                 360                 365
   Pro Thr Lys Leu Ser Ala Thr Ser Val Leu Tyr Tyr Asp Ser Ser Asn
       370                 375                 380
   Asn Val Ile Leu Arg Lys Ala Arg Asn Met Val Val Lys Ala Cys Gly
   385                 390                 395                 400
   Cys His
(2) INFORMATION FOR SEQ ID NO:7:
   (i) SEQUENCE CHARACTERISTICS:
      (A) LENGTH: 1926 base pairs
      (B) TYPE: nucleic acid
      (C) STRANDEDNESS: single
      (D) TOPOLOGY: linear
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 93..1289
      (D) OTHER INFORMATION: /function= "OSTEOGENIC PROTEIN"
         /product= "mOP2-PP"
         /note= "mOP2 cDNA"
   (xi) SEQUENCE DESCRIPTION: SEQ ID NO:7:
   GCCAGGCACA GGTGCGCCGT CTGGTCCTCC CCGTCTGGCG TCAGCCGAGC CCGACCAGCT      60
   ACCAGTGGAT GCGCGCCGGC TGAAAGTCCG AG ATG GCT ATG CGT CCC GGG CCA       113
                                       Met Ala Met Arg Pro Gly Pro
                                        1               5
   CTC TGG CTA TTG GGC CTT GCT CTG TGC GCG CTG GGA GGC GGC CAC GGT       161
   Leu Trp Leu Leu Gly Leu Ala Leu Cys Ala Leu Gly Gly Gly His Gly
            10                  15                  20
   CCG CGT CCC CCG CAC ACC TGT CCC CAG CGT CGC CTG GGA GCG CGC GAG       209
   Pro Arg Pro Pro His Thr Cys Pro Gln Arg Arg Leu Gly Ala Arg Glu
        25                  30                  35
   CGC CGC GAC ATG CAG CGT GAA ATC CTG GCG GTG CTC GGG CTA CCG GGA       257
   Arg Arg Asp Met Gln Arg Glu Ile Leu Ala Val Leu Gly Leu Pro Gly
    40                  45                  50                  55
   CGG CCC CGA CCC CGT GCA CAA CCC GCC GCT GCC CGG CAG CCA GCG TCC       305
   Arg Pro Arg Pro Arg Ala Gln Pro Ala Ala Ala Arg Gln Pro Ala Ser
                    60                  65                  70
   GCG CCC CTC TTC ATG TTG GAC CTA TAC CAC GCC ATG ACC GAT GAC GAC       353
   Ala Pro Leu Phe Met Leu Asp Leu Tyr His Ala Met Thr Asp Asp Asp
                75                  80                  85
   GAC GGC GGG CCA CCA CAG GCT CAC TTA GGC CGT GCC GAC CTG GTC ATG       401
   Asp Gly Gly Pro Pro Gln Ala His Leu Gly Arg Ala Asp Leu Val Met
            90                  95                 100
   AGC TTC GTC AAC ATG GTG GAA CGC GAC CGT ACC CTG GGC TAC CAG GAG       449
   Ser Phe Val Asn Met Val Glu Arg Asp Arg Thr Leu Gly Tyr Gln Glu
       105                 110                 115
   CCA CAC TGG AAG GAA TTC CAC TTT GAC CTA ACC CAG ATC CCT GCT GGG       497
   Pro His Trp Lys Glu Phe His Phe Asp Leu Thr Gln Ile Pro Ala Gly
   120                 125                 130                 135
   GAG GCT GTC ACA GCT GCT GAG TTC CGG ATC TAC AAA GAA CCC AGC ACC      545
   Glu Ala Val Thr Ala Ala Glu Phe Arg Ile Tyr Lys Glu Pro Ser Thr
                   140                 145                 150
   CAC CCG CTC AAC ACA ACC CTC CAC ATC AGC ATG TTC GAA GTG GTC CAA      593
   His Pro Leu Asn Thr Thr Leu His Ile Ser Met Phe Glu Val Val Gln
               155                 160                 165
   GAG CAC TCC AAC AGG GAG TCT GAC TTG TTC TTT TTG GAT CTT CAG ACG      641
   Glu His Ser Asn Arg Glu Ser Asp Leu Phe Phe Leu Asp Leu Gln Thr
           170                 175                 180
   CTC CGA TCT GGG GAC GAG GGC TGG CTG GTG CTG GAC ATC ACA GCA GCC      689
   Leu Arg Ser Gly Asp Glu Gly Trp Leu Val Leu Asp Ile Thr Ala Ala
       185                 190                 195
   AGT GAC CGA TGG CTG CTG AAC CAT CAC AAG GAC CTG GGA CTC CGC CTC      737
   Ser Asp Arg Trp Leu Leu Asn His His Lys Asp Leu Gly Leu Arg Leu
   200                 205                 210                 215
   TAT GTG GAA ACC GCG GAT GGG CAC AGC ATG GAT CCT GGC CTG GCT GGT      785
   Tyr Val Glu Thr Ala Asp Gly His Ser Met Asp Pro Gly Leu Ala Gly
                   220                 225                 230
   CTG CTT GGA CGA CAA GCA CCA CGC TCC AGA CAG CCT TTC ATG GTA ACC      833
   Leu Leu Gly Arg Gln Ala Pro Arg Ser Arg Gln Pro Phe Met Val Thr
               235                 240                 245
   TTC TTC AGG GCC AGC CAG AGT CCT GTG CGG GCC CCT CGG GCA GCG AGA      881
   Phe Phe Arg Ala Ser Gln Ser Pro Val Arg Ala Pro Arg Ala Ala Arg
           250                 255                 260
   CCA CTG AAG AGG AGG CAG CCA AAG AAA ACG AAC GAG CTT CCG CAC CCC      929
   Pro Leu Lys Arg Arg Gln Pro Lys Lys Thr Asn Glu Leu Pro His Pro
       265                 270                 275
   AAC AAA CTC CCA GGG ATC TTT GAT GAT GGC CAC GGT TCC CGC GGC AGA      977
   Asn Lys Leu Pro Gly Ile Phe Asp Asp Gly His Gly Ser Arg Gly Arg
   280                 285                 290                 295
   GAG GTT TGC CGC AGG CAT GAG CTC TAC GTC AGC TTC CGT GAC CTT GGC     1025
   Glu Val Cys Arg Arg His Glu Leu Tyr Val Ser Phe Arg Asp Leu Gly
                   300                 305                 310
   TGG CTG GAC TGG GTC ATC GCC CCC CAG GGC TAC TCT GCC TAT TAC TGT     1073
   Trp Leu Asp Trp Val Ile Ala Pro Gln Gly Tyr Ser Ala Tyr Tyr Cys
               315                 320                 325
   GAG GGG GAG TGT GCT TTC CCA CTG GAC TCC TGT ATG AAC GCC ACC AAC      1121
   Glu Gly Glu Cys Ala Phe Pro Leu Asp Ser Cys Met Asn Ala Thr Asn
           330                 335                 340
   CAT GCC ATC TTG CAG TCT CTG GTG CAC CTG ATG AAG CCA GAT GTT GTC      1169
   His Ala Ile Leu Gln Ser Leu Val His Leu Met Lys Pro Asp Val Val
       345                 350                 355
   CCC AAG GCA TGC TGT GCA CCC ACC AAA CTG AGT GCC ACC TCT GTG CTG      1217
   Pro Lys Ala Cys Cys Ala Pro Thr Lys Leu Ser Ala Thr Ser Val Leu
   360                 365                 370                 375
   TAC TAT GAC AGC AGC AAC AAT GTC ATC CTG CGT AAA CAC CGT AAC ATG      1265
   Tyr Tyr Asp Ser Ser Asn Asn Val Ile Leu Arg Lys His Arg Asn Met
                   380                 385                 390
   GTG GTC AAG GCC TGT GGC TGC CAC TGAGGCCCCG CCCAGCATCC TGCTTCTACT     1319
   Val Val Lys Ala Cys Gly Cys His
               395
   ACCTTACCAT CTGGCCGGGC CCCTCTCCAG AGGCAGAAAC CCTTCTATGT TATCATAGCT    1379
   CAGACAGGGG CAATGGGAGG CCCTTCACTT CCCCTGGCCA CTTCCTGCTA AAATTCTGGT    1439
   CTTTCCCAGT TCCTCTGTCC TTCATGGGGT TTCGGGGCTA TCACCCCGCC CTCTCCATCC    1499
   TCCTACCCCA AGCATAGACT GAATGCACAC AGCATCCCAG AGCTATGCTA ACTGAGAGGT    1559
   CTGGGGTCAG CACTGAAGGC CCACATGAGG AAGACTGATC CTTGGCCATC CTCAGCCCAC    1619
   AATGGCAAAT TCTGGATGGT CTAAGAAGGC CCTGGAATTC TAAACTAGAT GATCTGGGCT    1679
   CTCTGCACCA TTCATTGTGG CAGTTGGGAC ATTTTTAGGT ATAACAGACA CATACACTTA    1739
   GATCAATGCA TCGCTGTACT CCTTGAAATC AGAGCTAGCT TGTTAGAAAA AGAATCAGAG    1799
   CCAGGTATAG CGGTGCATGT CATTAATCCC AGCGCTAAAG AGACAGAGAC AGGAGAATCT    1859
   CTGTGAGTTC AAGGCCACAT AGAAAGAGCC TGTCTCGGGA GCAGGAAAAA AAAAAAAAAC    1919
   GGAATTC                                                              1926
(2) INFORMATION FOR SEQ ID NO:8:
   (i) SEQUENCE CHARACTERISTICS:
      (A) LENGTH: 399 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Ala Met Arg Pro Gly Pro Leu Trp Leu Leu Gly Leu Ala Leu Cys
    1               5                  10                  15
   Ala Leu Gly Gly Gly His Gly Pro Arg Pro Pro His Thr Cys Pro Gln
               20                  25                  30
   Arg Arg Leu Gly Ala Arg Glu Arg Arg Asp Met Gln Arg Glu Ile Leu
           35                  40                  45
   Ala Val Leu Gly Leu Pro Gly Arg Pro Arg Pro Arg Ala Gln Pro Ala
       50                  55                  60
   Ala Ala Arg Gln Pro Ala Ser Ala Pro Leu Phe Met Leu Asp Leu Tyr
   65                  70                  75                  80
   His Ala Met Thr Asp Asp Asp Asp Gly Gly Pro Pro Gln Ala His Leu
                   85                  90                  95
   Gly Arg Ala Asp Leu Val Met Ser Phe Val Asn Met Val Glu Arg Asp
               100                 105                 110
   Arg Thr Leu Gly Tyr Gln Glu Pro His Trp Lys Glu Phe His Phe Asp
           115                 120                 125
   Leu Thr Gln Ile Pro Ala Gly Glu Ala Val Thr Ala Ala Glu Phe Arg
       130                 135                 140
   Ile Tyr Lys Glu Pro Ser Thr His Pro Leu Asn Thr Thr Leu His Ile
   145                 150                 155                 160
   Ser Met Phe Glu Val Val Gln Glu His Ser Asn Arg Glu Ser Asp Leu
                   165                 170                 175
   Phe Phe Leu Asp Leu Gln Thr Leu Arg Ser Gly Asp Glu Gly Trp Leu
               180                 185                 190
   Val Leu Asp Ile Thr Ala Ala Ser Asp Arg Trp Leu Leu Asn His His
           195                 200                 205
   Lys Asp Leu Gly Leu Arg Leu Tyr Val Glu Thr Ala Asp Gly His Ser
       210                 215                 220
   Met Asp Pro Gly Leu Ala Gly Leu Leu Gly Arg Gln Ala Pro Arg Ser
   225                 230                 235                 240
   Arg Gln Pro Phe Met Val Thr Phe Phe Arg Ala Ser Gln Ser Pro Val
   245 250 255
   Arg Ala Pro Arg Ala Ala Arg Pro Leu Lys Arg Arg Gln Pro Lys Lys
   260 265 270
   Thr Asn Glu Leu Pro His Pro Asn Lys Leu Pro Gly Ile Phe Asp Asp
   275 280 285
   Gly His Gly Ser Arg Gly Arg Glu Val Cys Arg Arg His Glu Leu Tyr
   290 295 300
   Val Ser Phe Arg Asp Leu Gly Trp Leu Asp Trp Val Ile Ala Pro Gln
   305 310 315 320
   Gly Tyr Ser Ala Tyr Tyr Cys Glu Gly Glu Cys Ala Phe Pro Leu Asp
   325 330 335
   Ser Cys Met Asn Ala Thr Asn His Ala Ile Leu Gln Ser Leu Val His
   340 345 350
   Leu Met Lys Pro Asp Val Val Pro Lys Ala Cys Cys Ala Pro Thr Lys
   355 360 365
   Leu Ser Ala Thr Ser Val Leu Tyr Tyr Asp Ser Ser Asn Asn Val Ile
   370 375 380
   Leu Arg Lys His Arg Asn Met Val Val Lys Ala Cys Gly Cys His
   385 390 395
(2) INFORMATION FOR SEQ ID NO:9:
   (i) SEQUENCE CHARACTERISTICS:
      (A) LENGTH: 399 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..399
      (D) OTHER INFORMATION: /note= "PRE-PR0-OP3 (MOUSE)"
   (xi) SEQUENCE DESCRIPTION: SEQ ID NO:9:
   Met Ala Ala Arg Pro Gly Leu Leu Trp Leu Leu Gly Leu Ala Leu Cys
   1               5                   10                  15
   Val Leu Gly Gly Gly His Leu Ser His Pro Pro His Val Phe Pro Gln
               20                  25                  30
   Arg Arg Leu Gly Val Arg Glu Pro Arg Asp Met Gln Arg Glu Ile Arg
           35                  40                  45
   Glu Val Leu Gly Leu Ala Gly Arg Pro Arg Ser Arg Ala Pro Val Gly
       50                  55                  60
   Ala Ala Gln Gln Pro Ala Ser Ala Pro Leu Phe Met Leu Asp Leu Tyr
   65                  70                  75                  80
   Arg Ala Met Thr Asp Asp Ser Gly Gly Gly Thr Pro Gln Pro His Leu
                   85                  90                  95
   Asp Arg Ala Asp Leu Ile Met Ser Phe Val Asn Ile Val Glu Arg Asp
               100                 105                 110
   Arg Thr Leu Gly Tyr Gln Glu Pro His Trp Lys Glu Phe His Phe Asp
           115                 120                 125
   Leu Thr Gln Ile Pro Ala Gly Glu Ala Val Thr Ala Ala Glu Phe Arg
       130                 135                 140
   Ile Tyr Lys Glu Pro Ser Thr His Pro Leu Asn Thr Thr Leu His Ile
   145                 150                 155                 160
   Ser Met Phe Glu Val Val Gln Glu His Ser Asn Arg Glu Ser Asp Leu
                   165                 170                 175
   Phe Phe Leu Asp Leu Gln Thr Leu Arg Ser Gly Asp Glu Gly Trp Leu
               180                 185                 190
   Val Leu Asp Ile Thr Ala Ala Ser Asp Arg Trp Leu Leu Asn His His
           195                 200                 205
   Lys Asp Leu Gly Leu Arg Leu Tyr Val Glu Thr Glu Asp Gly His Ser
       210                 215                 220
   Ile Asp Pro Gly Leu Ala Gly Leu Leu Gly Arg Gln Ala Pro Arg Ser
   225                 230                 235                 240
   Arg Gln Pro Phe Met Val Gly Phe Phe Arg Ala Asn Gln Ser Pro Val
                   245                 250                 255
   Arg Ala Pro Arg Thr Ala Arg Pro Leu Lys Lys Lys Gln Leu Asn Gln
               260                 265                 270
   Ile Asn Gln Leu Pro His Ser Asn Lys His Leu Gly Ile Leu Asp Asp
           275                 280                 285
   Gly His Gly Ser His Gly Arg Glu Val Cys Arg Arg His Glu Leu Tyr
       290                 295                 300
   Val Ser Phe Arg Asp Leu Gly Trp Leu Asp Ser Val Ile Ala Pro Gln
   305                 310                 315                 320
   Gly Tyr Ser Ala Tyr Tyr Cys Ala Gly Glu Cys Ile Tyr Pro Leu Asn
                   325                 330                 335
   Ser Cys Met Asn Ser Thr Asn His Ala Thr Met Gln Ala Leu Val His
               340                 345                 350
   Leu Met Lys Pro Asp Ile Ile Pro Lys Val Cys Cys Val Pro Thr Glu
           355                 360                 365
   Leu Ser Ala Ile Ser Leu Leu Tyr Tyr Asp Arg Asn Asn Asn Val Ile
       370                 375                 380
   Leu Arg Arg Glu Arg Asn Met Val Val Gln Ala Cys Gly Cys His
   385                 390                 395
(2) INFORMATION FOR SEQ ID NO:10:
   (i) SEQUENCE CHARACTERISTICS:
      (A) LENGTH: 396 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..396
      (D) OTHER INFORMATION: /note= "PRE-PRO-BMP2 (HUMAN)"
   (x) PUBLICATION INFORMATION:
      (A) AUTHORS: WOZNEY,
      (C) JOURNAL: SCIENCE
      (D) VOLUME: 242
      (F) PAGES: 1528-1534
      (G) DATE: 1988
   (xi) SEQUENCE DESCRIPTION: SEQ ID NO:10:
(2) INFORMATION FOR SEQ ID NO:11:
   (i) SEQUENCE CHARACTERISTICS:
      (A) LENGTH: 408 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..408
      (D) OTHER INFORMATION: /note= "PRE-PRO-BMP4 (HUMAN)"
   (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 588 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..588
      (D) OTHER INFORMATION: /note= "PRE-PR0-DPP"
   (x) PUBLICATION INFORMATION:
      (A) AUTHORS: PADGETT,
      (C) JOURNAL: NATURE
      (D) VOLUME: 325
      (F) PAGES: 81-84
      (G) DATE: 1987
   (xi) SEQUENCE DESCRIPTION: SEQ ID NO:12:
   Met Arg Ala Trp Leu Leu Leu Leu Ala Val Leu Ala Thr Phe Gln Thr
   1 5 10 15
   Ile Val Arg Val Ala Ser Thr Glu Asp Ile Ser Gln Arg Phe Ile Ala
   20 25 30
   Ala Ile Ala Pro Val Ala Ala His Ile Pro Leu Ala Ser Ala Ser Gly
   35 40 45
   Ser Gly Ser Gly Arg Ser Gly Ser Arg Ser Val Gly Ala Ser Thr Ser
   50 55 60
   Thr Ala Leu Ala Lys Ala Phe Asn Pro Phe Ser Glu Pro Ala Ser Phe
   65 70 75 80
   Ser Asp Ser Asp Lys Ser His Arg Ser Lys Thr Asn Lys Lys Pro Ser
   85 90 95
   Lys Ser Asp Ala Asn Arg Gln Phe Asn Glu Val His Lys Pro Arg Thr
   100 105 110
   Asp Gln Leu Glu Asn Ser Lys Asn Lys Ser Lys Gln Leu Val Asn Lys Pro
   115 120 125
   Asn His Asn Lys Met Ala Val Lys Glu Gln Arg Ser His His Lys Lys
   130 135 140 145
   Ser His His His Arg Ser His Gln Pro Lys Gln Ala Ser Ala Ser Thr
   150 155 160
   Glu Ser His Gln Ser Ser Ser Ile Glu Ser Ile Phe Val Glu Glu Pro
   165 170 175
   Thr Leu Val Leu Asp Arg Glu Val Ala Ser Ile Asn Val Pro Ala Ser
   180 185 190
   Ala Lys Ala Ile Ile Ala Glu Gln Gly Pro Ser Thr Tyr Ser Lys Glu
   195 200 205
   Ala Leu Ile Lys Asp Lys Leu Lys Pro Asp Pro Ser Thr Leu Val Glu
   210 215 220 225
   Ile Glu Lys Ser Leu Leu Ser Leu Phe Asn Met Lys Arg Pro Pro Lys
   230 235 240
   Ile Asp Arg Ser Lys Ile Ile Ile Pro Glu Pro Met Lys Lys Leu Tyr
   245 250 255
   Ala Glu Ile Met Gly His Glu Leu Asp Ser Val Asn Ile Pro Lys Pro
   260 265 270
   Gly Leu Leu Thr Lys Ser Ala Asn Thr Val Arg Ser Phe Thr His Lys
   275 280 285
   Asp Ser Lys Ile Asp Asp Arg Phe Pro His His His Arg Phe Arg Leu
   290 295 300 305
   His Phe Asp Val Lys Ser Ile Pro Ala Asp Glu Lys Leu Lys Ala Ala
   310 315 320
   Glu Leu Gln Leu Thr Arg Asp Ala Leu Ser Gln Gln Val Val Ala Ser
   325 330 335
   Arg Ser Ser Ala Asn Arg Thr Arg Tyr Gln Val Leu Val Tyr Asp Ile
   340 345 350
   Thr Arg Val Gly Val Arg Gly Gln Arg Glu Pro Ser Tyr Leu Leu Leu
   355 360 365
   Asp Thr Lys Thr Val Arg Leu Asn Ser Thr Asp Thr Val Ser Leu Asp
   370 375 380 385
   Val Gln Pro Ala Val Asp Arg Trp Leu Ala Ser Pro Gln Arg Asn Tyr
   390 395 400
   Gly Leu Leu Val Glu Val Arg Thr Val Arg Ser Leu Lys Pro Ala Pro
   405 410 415
   His His His Val Arg Leu Arg Arg Ser Ala Asp Glu Ala His Glu Arg
   420 425 430
   Trp Gln His Lys Gln Pro Leu Leu Phe Thr Tyr Thr Asp Asp Gly Arg
   435 440 445
   His Lys Ala Arg Ser Ile Arg Asp Val Ser Gly Gly Glu Gly Gly Gly
   450 455 460 465
   Lys Gly Gly Arg Asn Lys Arg His Ala Arg Arg Pro Thr Arg Arg Lys
   470 475 480
   Asn His Asp Asp Thr Cys Arg Arg His Ser Leu Tyr Val Asp Phe Ser
   485 490 495
   Asp Val Gly Trp Asp Asp Trp Ile Val Ala Pro Leu Gly Tyr Asp Ala
   500 505 510
   Tyr Tyr Cys His Gly Lys Cys Pro Phe Pro Leu Ala Asp His Phe Asn
   515 520 525
   Ser Thr Asn His Ala Val Val Gln Thr Leu Val Asn Asn Met Asn Pro
   530 535 540 545
   Gly Lys Val Pro Lys Ala Cys Cys Val Pro Thr Gln Leu Asp Ser Val
   550 555 560
   Ala Met Leu Tyr Leu Asn Asp Gln Ser Thr Val Val Leu Lys Asn Tyr
   565 570 575
   Gln Glu Met Thr Val Val Gly Cys Gly Cys Arg
   580 585
(2) INFORMATION FOR SEQ ID NO:13:
   (i) SEQUENCE CHARACTERISTICS:
      (A) LENGTH: 359 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..359
      (D) OTHER INFORMATION: /note= "PRE-PR0-VG1"
   (x) PUBLICATION INFORMATION:
      (A) AUTHORS: WEEKS,
      (C) JOURNAL: CELL
      (D) VOLUME: 51
      (F) PAGES: 861-867
      (G) DATE: 1987
   (xi) SEQUENCE DESCRIPTION: SEQ ID NO:13:
(2) INFORMATION FOR SEQ ID NO:14:
   (i) SEQUENCE CHARACTERISTICS:
      (A) LENGTH: 438 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..438
      (D) OTHER INFORMATION: /note= "PRE-PR0-VGR1"
   (x) PUBLICATION INFORMATION:
      (A) AUTHORS: LYONS,
      (C) JOURNAL: Proc. Natl. Acad. Sci. U.S.A.
      (D) VOLUME: 86
      (F) PAGES: 4554-4558
      (G) DATE: 1989
   (xi) SEQUENCE DESCRIPTION: SEQ ID NO:14:
   Met Arg Lys Met Gln Lys Glu Ile Leu Ser Val Leu Gly Pro Pro His
   1 5 10 15
   Arg Pro Arg Pro Leu His Gly Leu Gln Gln Pro Gln Pro Pro Val Leu
   20 25 30
   Pro Pro Gln Gln Gln Gln Gln Gln Gln Gln Gln Gln Thr Ala Asp Glu
   35 40 45
   Glu Pro Pro Pro Gly Arg Leu Lys Ser Ala Pro Leu Phe Met Leu Asp
   50 55 60
   Leu Tyr Asn Ala Leu Ser Asn Asp Asp Glu Glu Asp Gly Ala Ser Glu
   65                  70                  75                  80
   Gly Val Gly Gln Glu Pro Gly Ser His Gly Gly Ala Ser Ser Ser Gln
                   85                  90                  95
   Leu Arg Gln Pro Ser Pro Gly Ala Ala His Ser Leu Asn Arg Lys Ser
               100                 105                 110
   Leu Leu Ala Pro Gly Pro Gly Gly Gly Ala Ser Pro Leu Thr Ser Ala
           115                 120                 125
   Gln Asp Ser Ala Phe Leu Asn Asp Ala Asp Met Val Met Ser Phe Val
       130                 135                 140
   Asn Leu Val Gly Tyr Asp Lys Glu Phe Ser Pro His Gln Arg His His
   145                 150                 155                 160
   Lys Glu Phe Lys Phe Asn Leu Ser Gln Ile Pro Glu Gly Glu Ala Val
                   165                 170                 175
   Thr Ala Ala Glu Phe Arg Val Tyr Lys Asp Cys Val Val Gly Ser Phe
               180                 185                 190
   Lys Asn Gln Thr Phe Leu Ile Ser Ile Tyr Gln Val Leu Gln Glu Ala
           195                 200                 205
   Gln His Arg Asp Ser Asp Leu Phe Leu Leu Asp Thr Arg Val Val Trp
       210                 215                 220
   Ala Ser Glu Glu Gly Trp Leu Glu Phe Asp Ile Thr Ala Thr Ser Asn
   225                 230                 235                 240
   Leu Trp Val Val Ile Pro Gln His Asn Met Gly Leu Gln Leu Ser Val
                   245                 250                 255
   Val Thr Arg Asp Gly Leu His Val Asn Pro Arg Ala Ala Gly Leu Val
               260                 265                 270
   Gly Arg Asp Gly Pro Tyr Asp Lys Gln Pro Phe Met Val Ala Phe Phe
           275                 280                 285
   Lys Val Ser Glu Val His Val Arg Ser Thr Arg Ser Ala Ser Ser Arg
       290                 295                 300
   Arg Arg Gln Gln Ser Arg Asn Arg Ser Thr Gln Ser Gln Asp Val Ser
   305                 310                 315                 320
   Arg Gly Ser Gly Ser Ser Asp Tyr Asn Gly Ser Glu Leu Lys Thr Ala
                   325                 330                 335
   Cys Lys Lys His Glu Leu Tyr Val Ser Phe Gln Asp Leu Gly Trp Gln
   340 345 350
   Asp Trp Ile Ile Ala Pro Lys Gly Tyr Ala Ala Asn Tyr Cys Asp Gly
   355 360 365
   Glu Cys Ser Phe Pro Leu Asn Ala His Met Asn Ala Thr Asn His Ala
   370 375 380
   Ile Val Gln Thr Leu Val His Leu Met Asn Pro Glu Thr Val Pro Lys
   385 390 395 400
   Pro Cys Cys Ala Pro Thr Lys Leu Asn Ala Ile Ser Val Leu Tyr Phe
   405 410 415
   Asp Asp Asn Ser Asn Val Ile Leu Lys Lys Tyr Arg Asn Met Val Val
   420 425 430
   Arg Ala Cys Gly Cys His
   435
(2) INFORMATION FOR SEQ ID NO:15:
   (i) SEQUENCE CHARACTERISTICS:
      (A) LENGTH: 372 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..372
      (D) OTHER INFORMATION: /note= "PRE-PR0-GDF-1"
   (x) PUBLICATION INFORMATION:
      (A) AUTHORS: LEE,
      (B) TITLE: EXPRESSION OF GROVTH/DIFFERENTIATION FACTOR 1
      (C) JOURNAL: Proc. Natl. Acad. Sci. U.S.A.
      (D) VOLUME: 88
      (F) PAGES: 4250-4254
      (G) DATE: MAY-1991
   (xi) SEQUENCE DESCRIPTION: SEQ ID NO:15:
   Met Pro Pro Pro Gln Gln Gly Pro Cys Gly His His Leu Leu Leu Leu
   1               5                   10                  15
   Leu Ala Leu Leu Leu Pro Ser Leu Pro Leu Thr Arg Ala Pro Val Pro
               20                  25                  30
   Pro Gly Pro Ala Ala Ala Leu Leu Gln Ala Leu Gly Leu Arg Asp Glu
           35                  40                  45
   Pro Gln Gly Ala Pro Arg Leu Arg Pro Val Pro Pro Val Met Trp Arg
       50                  55                  60
   Leu Phe Arg Arg Arg Asp Pro Gln Glu Thr Arg Ser Gly Ser Arg Arg
   65                  70                  75                  80
   Thr Ser Pro Gly Val Thr Leu Gln Pro Cys His Val Glu Glu Leu Gly
                   85                  90                  95
   Val Ala Gly Asn Ile Val Arg His Ile Pro Asp Arg Gly Ala Pro Thr
               100                 105                 110
   Arg Ala Ser Glu Pro Val Ser Ala Ala Gly His Cys Pro Glu Trp Thr
           115                 120                 125
   Val Val Phe Asp Leu Ser Ala Val Glu Pro Ala Glu Arg Pro Ser Arg
       130                 135                 140
   Ala Arg Leu Glu Leu Arg Phe Ala Ala Ala Ala Ala Ala Ala Pro Glu
   145                 150                 155                 160
   Gly Gly Trp Glu Leu Ser Val Ala Gln Ala Gly Gln Gly Ala Gly Ala
                   165                 170                 175
   Asp Pro Gly Pro Val Leu Leu Arg Gln Leu Val Pro Ala Leu Gly Pro
               180                 185                 190
   Pro Val Arg Ala Glu Leu Leu Gly Ala Ala Trp Ala Arg Asn Ala Ser
           195                 200                 205
   Trp Pro Arg Ser Leu Arg Leu Ala Leu Ala Leu Arg Pro Arg Ala Pro
       210                 215                 220
   Ala Ala Cys Ala Arg Leu Ala Glu Ala Ser Leu Leu Leu Val Thr Leu
   225                 230                 235                 240
   Asp Pro Arg Leu Cys His Pro Leu Ala Arg Pro Arg Arg Asp Ala Glu
                   245                 250                 255
   Pro Val Leu Gly Gly Gly Pro Gly Gly Ala Cys Arg Ala Arg Arg Leu
               260                 265                 270
   Tyr Val Ser Phe Arg Glu Val Gly Trp His Arg Trp Val Ile Ala Pro
   275 280 285
   Arg Gly Phe Leu Ala Asn Tyr Cys Gln Gly Gln Cys Ala Leu Pro Val
   290 295 300
   Ala Leu Ser Gly Ser Gly Gly Pro Pro Ala Leu Asn His Ala Val Leu
   305 310 315 320
   Arg Ala Leu Met His Ala Ala Ala Pro Gly Ala Ala Asp Leu Pro Cys
   325 330 335
   Cys Val Pro Ala Arg Leu Ser Pro Ile Ser Val Leu Phe Phe Asp Asn
   340 345 350
   Ser Asp Asn Val Val Leu Arg Gln Tyr Glu Asp Met Val Val Asp Glu
   355 360 365
   Cys Gly Cys Arg
   370
(2) INFORMATION FOR SEQ ID NO:16:
   (i) SEQUENCE CHARACTERISTICS:
      (A) LENGTH: 455 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..455
      (D) OTHER INFORMATION: /note= "PRE-PRO 60A"
   (x) PUBLICATION INFORMATION:
      (A) AUTHORS: WHARTON,
      (C) JOURNAL: Proc. Natl. Acad. Sci. U.S.A.
      (D) VOLUME: 88
      (F) PAGES: 9214-9218
      (G) DATE: 1991
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 472 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..472
      (D) OTHER INFORMATION: /note= "PAE-PR0-BHF3"
   (x) PUBLICATION INFORMATION:
      (A) AUTHORS: WOZNEY,
      (C) JOURNAL: SCIENCE
      (D) VOLUME: 242
      (F) PAGES: 1528-1534
      (G) DATE: 1988
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 453 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..453
      (D) OTHER INFORMATION: /note= "PRE-PRO-BMP5 (HUMAN)"
   (x) PUBLICATION INFORMATION:
      (A) AUTHORS: CELESTE,
      (C) JOURNAL: Proc. Natl. Acad. Sci. U.S.A.
      (D) VOLUME: 87
      (F) PAGES: 9843-9847
      (G) DATE: 1991
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 513 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..513
      (D) OTHER INFORMATION: /note= "PRE-PRO-BMP6 (HUMAN)"
   (x) PUBLICATION INFORMATION:
      (A) AUTHORS: CELESTE,
      (C) JOURNAL: Proc. Natl. Acad. Sci. U.S.A.
      (D) VOLUME: 87
      (F) PAGES: 9843-9847
      (G) DATE: 1991
   (xi) SEQUENCE DESCRIPTION: SEQ ID NO:19:
   Met Pro Gly Leu Gly Arg Arg Ala Gln Trp Leu Cys Trp Trp Trp Gly
   1 5 10 15
   Leu Leu Cys Ser Cys Cys Gly Pro Pro Pro Leu Arg Pro Pro Leu Pro
   20 25 30
   Ala Ala Ala Ala Ala Ala Ala Gly Gly Gln Leu Leu Gly Asp Gly Gly
   35 40 45
   Ser Pro Gly Arg Thr Glu Gln Pro Pro Pro Ser Pro Gln Ser Ser Ser
   50 55 60
   Gly Phe Leu Tyr Arg Arg Leu Lys Thr Gln Glu Lys Arg Glu Met Gln
   65 70 75 80
   Lys Glu Ile Leu Ser Val Leu Gly Leu Pro His Arg Pro Arg Pro Leu
   85 90 95
   His Gly Leu Gln Gln Pro Gln Pro Pro Ala Leu Arg Gln Gln Glu Glu
   100 105 110
   Gln Gln Gln Gln Gln Gln Leu Pro Arg Gly Glu Pro Pro Pro Gly Arg
   115 120 125
   Leu Lys Ser Ala Pro Leu Phe Met Leu Asp Leu Tyr Asn Ala Leu Ser
   130 135 140
   Ala Asp Asn Asp Glu Asp Gly Ala Ser Glu Gly Glu Arg Gln Gln Ser
   145 150 155 160
   Trp Pro His Glu Ala Ala Ser Ser Ser Gln Arg Arg Gln Pro Pro Pro
   165 170 175
   Gly Ala Ala His Pro Leu Asn Arg Lys Ser Leu Leu Ala Pro Gly Ser
   180 185 190
   Gly Ser Gly Gly Ala Ser Pro Leu Thr Ser Ala Gln Asp Ser Ala Phe
   195 200 205
   Leu Asn Asp Ala Asp Met Val Met Ser Phe Val Asn Leu Val Glu Tyr
   210 215 220
   Asp Lys Glu Phe Ser Pro Arg Gln Arg His His Lys Glu Phe Lys Phe
   225 230 235 240
   Asn Leu Ser Gln Ile Pro Glu Gly Glu Val Val Thr Ala Ala Glu Phe
   245 250 255
   Arg Ile Val Lys Asp Cys Val Met Gly Ser Phe Lys Asn Gln Thr Phe
   260 265 270
   Leu Ile Ser Ile Tyr Gln Val Leu Gln Glu His Gln His Arg Asp Ser
   275 280 285
   Asp Leu Phe Leu Leu Asp Thr Arg Val Val Trp Ala Ser Glu Glu Gly
   290 295 300
   Trp Leu Glu Phe Asp Ile Thr Ala Thr Ser Asn Leu Trp Val Val Thr
   305 310 315 320
   Pro Gln His Asn Met Gly Leu Gln Leu Ser Val Val Thr Arg Asp Gly
                   325                 330                 335
   Val His Val His Pro Arg Ala Ala Gly Leu Val Gly Arg Asp Gly Pro
               340                 345                 350
   Tyr Asp Lys Gln Pro Phe Met Val Ala Phe Phe Lys Val Ser Glu
           355                 360                 365
   Val His Val Arg Thr Thr Arg Ser Ala Ser Ser Arg Arg Arg Gln Gln
           370                 375                 380
   Ser Arg Asn Arg Ser Thr Gln Ser Gln Asp Val Ala Arg Val Ser Ser
       385                 390                 395
   Ala Ser Asp Tyr Asn Ser Ser Glu Leu Lys Thr Ala Cys Arg Lys His
   400                 405                 410                     415
   Glu Leu Tyr Val Ser Phe Gln Asp Leu Gly Trp Gln Asp Trp Ile Ile
                   420                 425                 430
   Ala Pro Lys Gly Tyr Ala Ala Asn Tyr Cys Asp Gly Glu Cys Ser Phe
               435                 440                 445
   Pro Leu Asn Ala His Met Asn Ala Thr Asn His Ala Ile Val Gln Thr
           450                 455                 460
   Leu Val His Leu Met Asn Pro Glu Tyr Val Pro Lys Pro Cys Cys Ala Pro
       465                 470                 475                     480
   Thr Lys Leu Asn Ala Ile Ser Val Leu Tyr Phe Asp Asp Asn Ser Asn
                   485                 490                 495
   Val Ile Leu Lys Lys Tyr Arg Asn Met Val Val Arg Ala Cys Gly Cys
               500                 505                 510
   His
(2) INFORMATION FOR SEQ ID NO:20:
   (i) SEQUENCE CHARACTERISTICS:
      (A) LENGTH: 97 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..97
      (D) OTHER INFORMATION: /label= Generic-Seq-7
         /note= "vherein each Xaa is independently selected from a group of one or more specified amino acids as defined in the specification."
   (xi) SEQUENCE DESCRIPTION: SEQ ID NO:20:
   Leu Xaa Xaa Xaa Phe Xaa Xaa Xaa Gly Trp Xaa Xaa Xaa Xaa Xaa Xaa
   1               5                   10                  15
   Pro Xaa Xaa Xaa Xaa Ala Xaa Tyr Cys Xaa Gly Xaa Cys Xaa Xaa Pro
               20                  25                  30
   Xaa Xaa Xaa Xaa Xaa Xaa Xaa Xaa Asn His Ala Xaa Xaa Xaa Xaa Xaa
           35                  40                  45
   Xaa Xaa Xaa Xaa Xaa Xaa Xaa Xaa Xaa Xaa Xaa Xaa Cys Cys Xaa Pro
       50                  55                  60
   Xaa Xaa Xaa Xaa Xaa Xaa Xaa Xaa Leu Xaa Xaa Xaa Xaa Xaa Xaa Xaa
   65                  70                  75                  80
   Val Xaa Leu Xaa Xaa Xaa Xaa Xaa Met Xaa Val Xaa Xaa Cys Xaa Cys
                   85                  90                  95
   Xaa
(2) INFORMATION FOR SEQ ID NO:21:
   (i) SEQUENCE CHARACTERISTICS:
      (A) LENGTH: 102 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= Generic-Seq-8
         /note= "wherin each Xaa is independently selected from a group of one or more specified amino acids as defined in the specification."
   (xi) SEQUENCE DESCRIPTION: SEQ ID NO:21:
   Cys Xaa Xaa Xaa Xaa Leu Xaa Xaa Xaa Phe Xaa Xaa Xaa Gly Trp Xaa
   1               5                   10                  15
   Xaa Xaa Xaa Xaa Xaa Pro Xaa Xaa Xaa Xaa Ala Xaa Tyr Cys Xaa Gly
               20                  25                  30
   Xaa Cys Xaa Xaa Pro Xaa Xaa Xaa Xaa Xaa Xaa Xaa Xaa Asn His Ala
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Cys Cys Xaa Pro Xaa Xaa Xaa Xaa Xaa Xaa Xaa Xaa Leu Xaa Xaa
   65                  70                  75                  80
   Xaa Xaa Xaa Xaa Xaa Val Xaa Leu Xaa Xaa Xaa Xaa Xaa Met Xaa Val
                   85                  90                  95
   Xaa Xaa Cys Xaa Cys Xaa
               100
(2) INFORMATION FOR SEQ ID NO:22:
   (i) SEQUENCE CHARACTERISTICS:
      (A) LENGTH: 102 amino acids
      (B) TYPE: amino acid
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= OPX
         /note= "WHEREIN EACH XAA IS INDEPENDENTLY SELECTED FROM A GROUP OF ONE OR MORE SPECIFIED AMINO ACIDS AS DEFINED IN THE SPECIFICATION (SECTION II.B.2.)"
   (xi) SEQUENCE DESCRIPTION: SEQ ID NO:22:
   Cys Xaa Xaa His Glu Leu Tyr Val Xaa Phe Xaa Asp Leu Gly Trp Xaa
   1 5 10 15
   Asp Trp Xaa Ile Ala Pro Xaa Gly Tyr Xaa Ala Tyr Tyr Cys Glu Gly
   20 25 30
   Glu Cys Xaa Phe Pro Leu Xaa Ser Xaa Met Asn Ala Thr Asn His Ala
   35 40 45
   Ile Xaa Gln Xaa Leu Val His Xaa Xaa Xaa Pro Xaa Xaa Val Pro Lys
   50 55 60
   Xaa Cys Cys Ala Pro Thr Xaa Leu Xaa Ala Xaa Ser Val Leu Tyr Xaa
   65 70 75 80
   Asp Xaa Ser Xaa Asn Val Xaa Leu Xaa Lys Xaa Arg Asn Met Val Val
   85 90 95
   Xaa Ala Cys Gly Cys His
   100
(2) INFORMATION FOR SEQ ID NO:23:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Cleavage-site
      (B) LOCATION: 1..4
      (D) OTHER INFORMATION: /note= "PROTEOLYTIC CLEAVAGE SITE"
   (xi) SEQUENCE DESCRIPTION: SEQ ID NO:23:
   Arg Xaa Xaa Arg
   1